(19) Europäisches Patentamt
European Patent Office
Office européen des brevets

(11) EP 2 096 116 B1

(12) **EUROPEAN PATENT SPECIFICATION**

(45) Date of publication and mention of the grant of the patent:
**15.08.2012 Bulletin 2012/33**

(21) Application number: **07832309.4**

(22) Date of filing: **21.11.2007**

(51) Int Cl.:
***C07H 15/12*** *(2006.01)* ***A61K 47/26*** *(2006.01)*

(86) International application number:
**PCT/JP2007/072579**

(87) International publication number:
**WO 2008/062842 (29.05.2008 Gazette 2008/22)**

(54) **SODIUM SALT OF DISACCHARIDE COMPOUND, METHOD FOR PRODUCING THE SAME, AND USE OF THE SAME**

NATRIUMSALZ EINER DISACCHARIDVERBINDUNG, VERFAHREN ZU DEREN HERSTELLUNG UND DEREN VERWENDUNG

SEL DE SODIUM DE DISACCHARIDE ET SES MÉTHODES DE PRODUCTION ET D'UTILISATION

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **22.11.2006 US 860483 P**
**22.11.2006 JP 2006315020**

(43) Date of publication of application:
**02.09.2009 Bulletin 2009/36**

(73) Proprietor: **Eisai R&D Management Co., Ltd.**
**Bunkyo-Ku**
**Tokyo 112-8088 (JP)**

(72) Inventors:
- **SAKURAI, Shin**
**Kamisu-shi**
**Ibaraki 314-0255 (JP)**
- **FURUKAWA, Ken**
**Kamisu-shi**
**Ibaraki 314-0255 (JP)**
- **MATSUO, Kimihiro**
**Kamisu-shi**
**Ibaraki 314-0255 (JP)**
- **TAGAMI, Kenichi**
**Kamisu-shi**
**Ibaraki 314-0255 (JP)**

(74) Representative: **HOFFMANN EITLE**
**Patent- und Rechtsanwälte**
**Arabellastrasse 4**
**81925 München (DE)**

(56) References cited:
**JP-A- 2006 518 394** **US-A1- 2002 019 521**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 096 116 B1

## Description

### Field of the Invention

The present invention relates to a sodium salt of a disaccharide compound represented by the formula below:

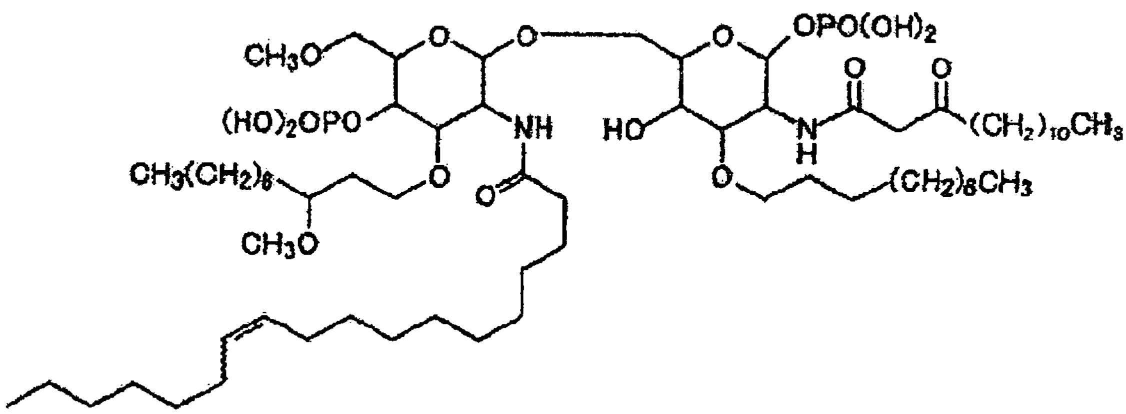

The present application claims priority rights based on Patent Application No. 2006-315020 filed in Japan, whose contents are cited herein.

### Background Art

The compounds represented by the above formula and tetrasodium salts thereof are known to have excellent effects in prevention and treatment of Gram-negative bacteremia, particularly endotoxin shock, in which there is a high mortality rate caused by endotoxins or lipopolysaccharide (LPS) components present in the outer membranes of Gram-negative bacteria. They have also been confirmed to have excellent anti-endotoxin effects in humans (see Lynn et al., J. Pharmacol. Exp. Ther. 308 (1): 175-181, 2004). They are also known to have an antagonistic effect against TLR4 (toll-like receptor 4), a receptor which recognizes the bacterial body component of bacilli (see WO 2004/071465, WO 96/39411). Based on these effects, the aforementioned compounds and tetrasodium salts thereof have been reported to be particularly effective as prevention and treatment agents of sepsis, endotoxemia, improvement of prognosis after coronary artery bypass graft surgery and so forth (see WO 96/39411, WO 2004/074303).

### Disclosure of the Invention

### Problems the Invention Is To Resolve

It was discovered, however, that the tetrasodium salts of the aforementioned compounds decompose slightly during storage depending on storage conditions, and over time they generate trace amounts of impurities. From the viewpoint of stability as a medicine, decomposition of sodium salts of the aforementioned compounds over time during storage should be avoided at all costs.

Thus, the present invention has the purpose of providing a medicine with excellent stability by suppressing decomposition and reducing the generation of impurities over time of sodium salts of the compounds represented by the above formula.

### Means of Resolving Problems

The above purpose is achieved by a sodium salt represented by average formula (I):

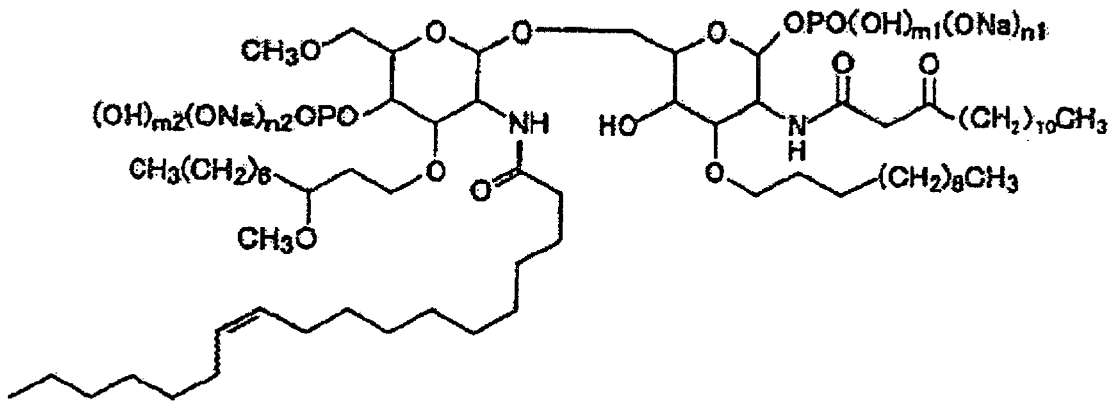

(in the formula, $m_1$, $n_1$, $m_2$ and $n_2$ independently represent 0 or a positive number not greater than 2, while satisfying $m_1 + n_1 = 2$, $m_2 + n_2 = 2$, $0 < m_1 + m_2 < 4$ and $0 < n_1 + n_2 < 4$).

In the above average formula (I), it is preferable if $3 \leq n_1 + n_2 < 4$, and additionally, it is more preferable if $3.5 \leq n_1 + n_2 \leq 3.8$.

Also, the sodium content of the sodium salt represented by the above average formula (I) is preferably at least 5.0 weight% and less than 6.5 weight%, more preferably at least 5.7 weight% and at most 6.3 weight%.

The sodium salt represented by average formula (I) can be obtained by incompletely neutralizing a compound represented by general formula (II):

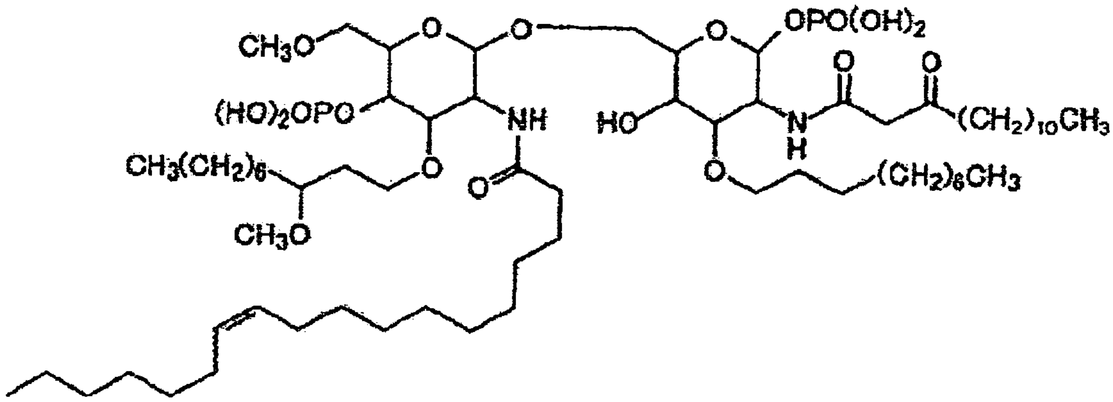

with a sodium-containing base.

Also, the purpose of the present invention can be achieved by a sodium salt represented by general formula (III):

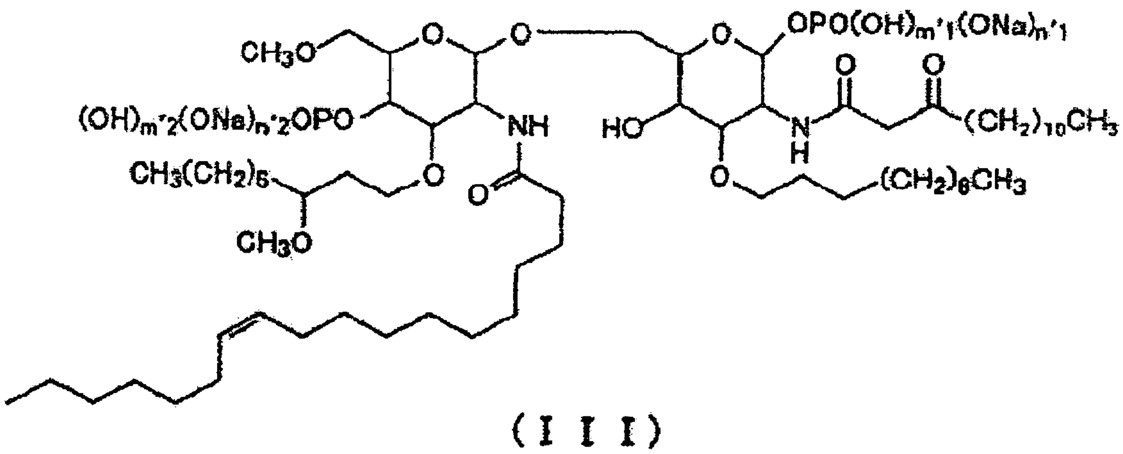

(III)

(in the formula, $m'_1$, $n'_1$, $m'_2$ and $n'_2$ independently represent an integer of 0 or 2 or less, while satisfying $m'_1 + n'_1 = 2$, $m'_2 + n'_2 = 2$, $0 < m'_1 + m'_2 < 4$ and $0 < n'_1 + n'_2 < 4$).

Specifically, by making a sodium salt represented by general formula (IV):

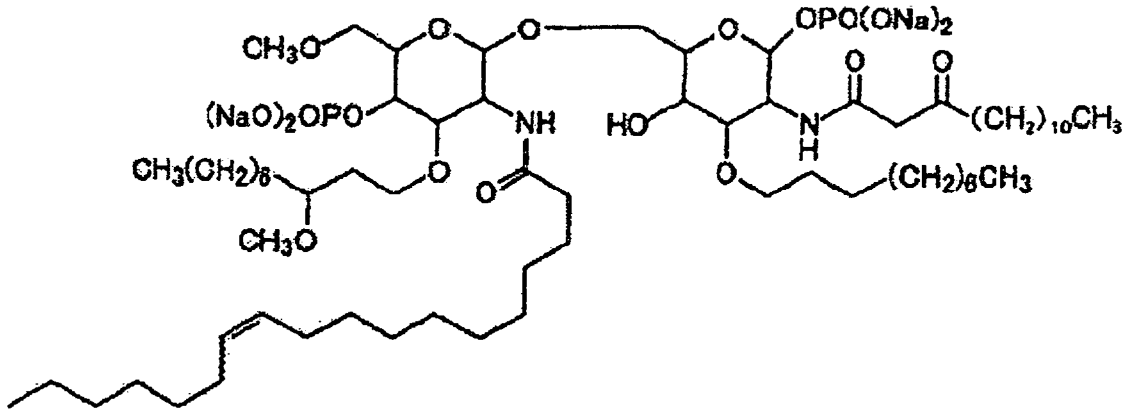

coexist with a sodium salt represented by general formula (III), decomposition of the sodium salt represented by general formula (IV) is suppressed, and generation of impurities in said salt is suppressed.

Therefore, the purpose of the present invention is achieved by making a sodium salt represented by the above general formula (IV) coexist with a sodium salt represented by general formula (III).

Effect of the Invention

In the sodium salt of the present invention represented by average formula (I), decomposition over time is strongly suppressed and stability is high, and therefore it has excellent stability as a medicine. The case where $3 \leq n_1 + n_2 < 4$, particularly $3.5 \leq n_1 + n_2 \leq 3.8$, in average formula (I), or the case where the sodium content is at least 5.0 weight% and less than 6.5 weight%, particularly at least 5.7 weight% and at most 6.3 weight%, are excellent.

The method of producing the sodium salt represented by average formula (I) of the present invention does not employ substances harmful to the human body and does not required a complex process, and therefore it is excellent as a method of producing medicine.

Additionally, the sodium salt represented by general formula (III) of the present invention suppresses decomposition and reduces generation of impurities over time of the sodium salt represented by general formula (IV), and therefore enables long-term storage of the sodium salt represented by general formula (IV).

Brief Explanation of the Drawings

FIG. 1 is a first diagram illustrating the impurity generation ratio of examples 2-4 and comparative example 1.
FIG. 2 is a second diagram illustrating the impurity generation ratio of examples 2-4 and comparative example 1.

Description of the Preferred Embodiments

The compounds represented by general formula (II) and the tetrasodium salts thereof represented by general formula (IV) related to the present invention can be synthesized by any known method.

For example, the compounds represented by general formula (IV) can be synthesized by the following process, as disclosed in WO96/39411.

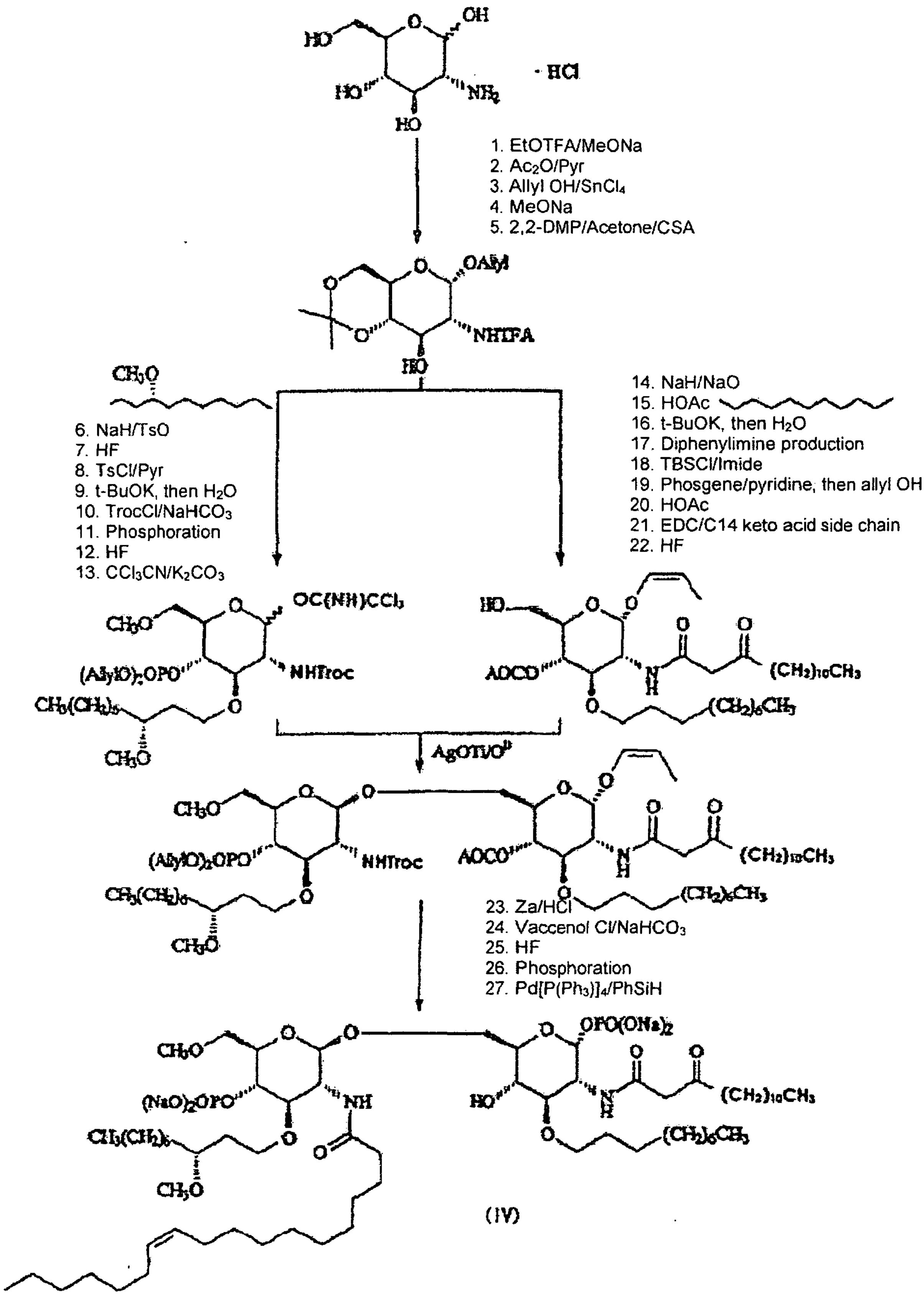


Additionally, the compounds represented by general formulas (II) and (IV) can be synthesized via scheme 1 below, as disclosed in WO2004/074303, in which two acyl-type side chains are first introduced into a monosaccharide, after which these are bonded, and via scheme 2, in which the compound obtained by scheme 1 is phosphorated.

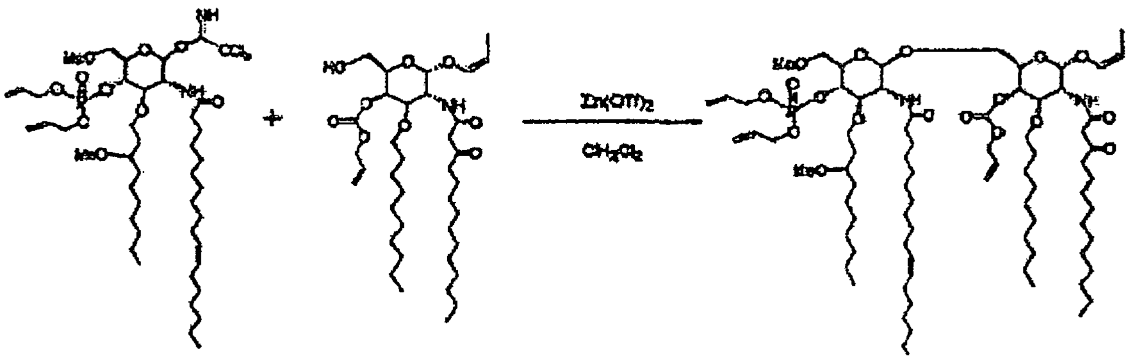


Scheme 1

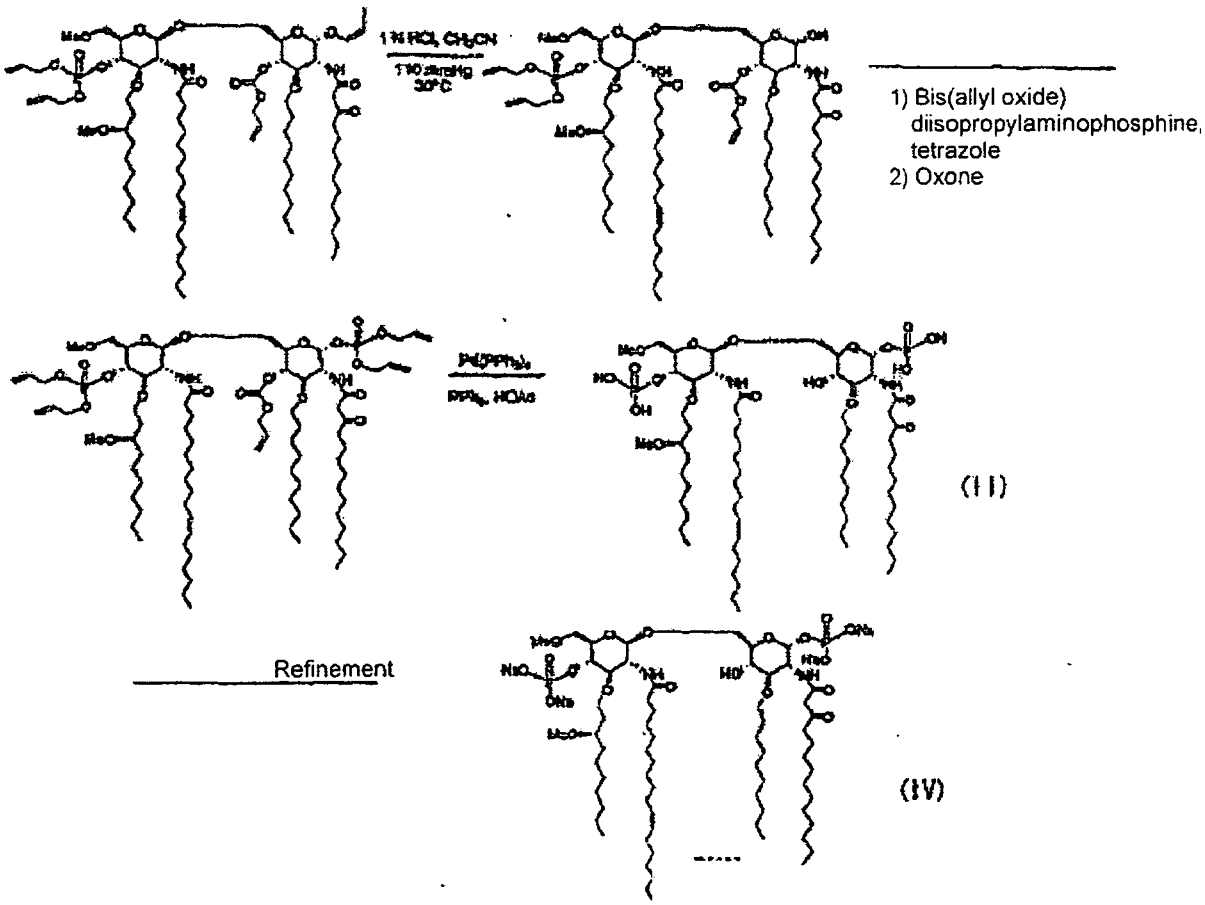


(II)

(IV)

Scheme 2

However, the method disclosed in WO96/39411 has the drawback in production efficiency that there are many synthesis steps. Additionally, the method of WO2004/074303, although there are many there synthesis steps, uses reagents which are toxic to the human body as well as reagents which are explosive, and therefore there is room for improvement from the viewpoint of safety and operationality.

Therefore, the compounds represented by general formulas (II) and (IV) are preferable in that they are produced by the method below, which has few synthesis steps and does not use reagents which are toxic to the human body or reagents which are explosive.

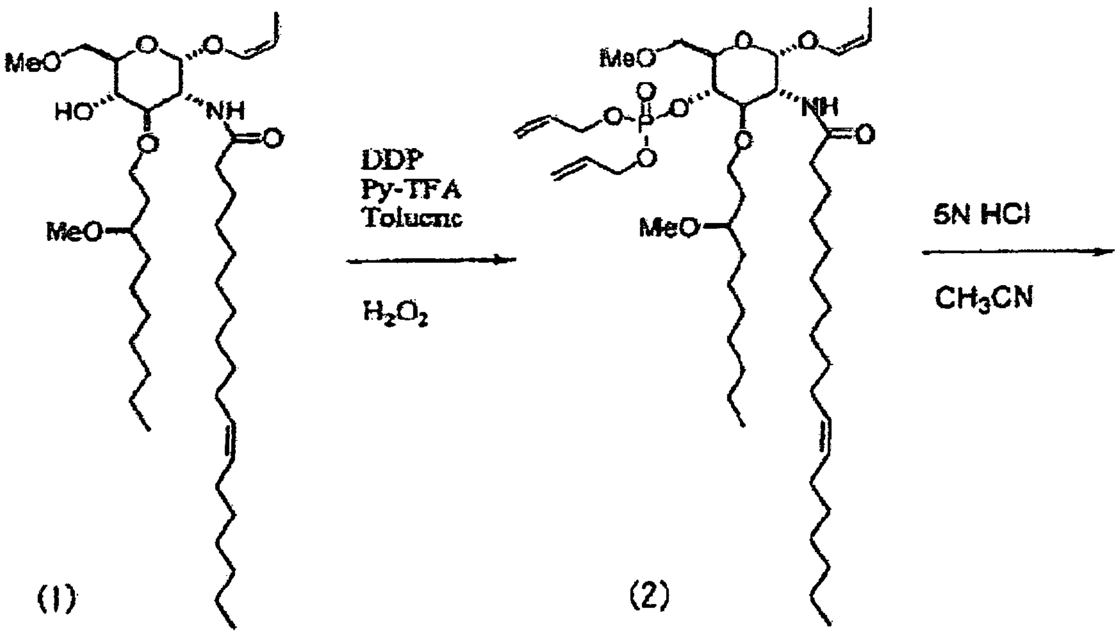


DDP: Diallyl N,N-diisopropyl phosphoramidate

Py: Pyridine

TFA: Trifluoroacetic acid

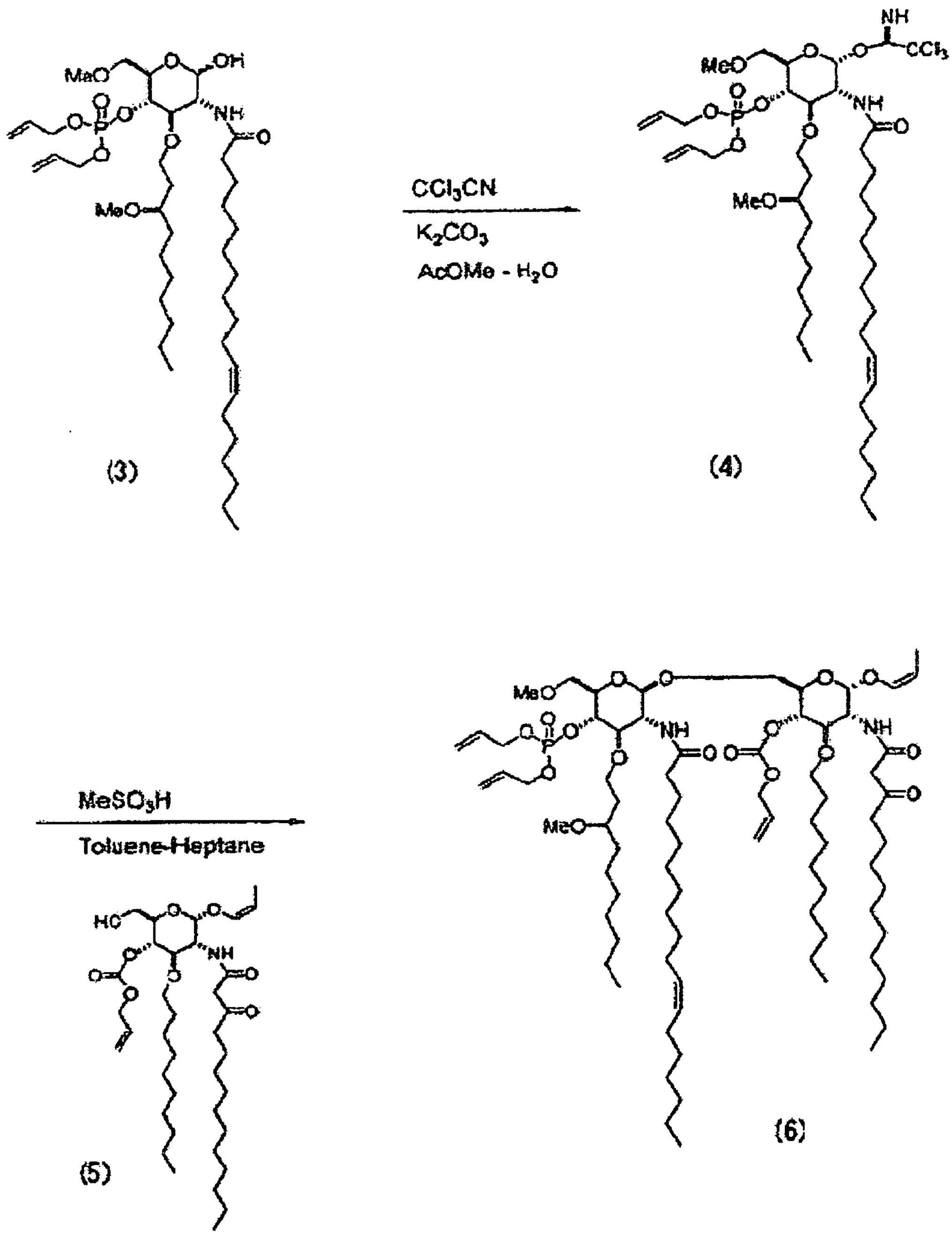


(3)

(4)

(5)

(6)

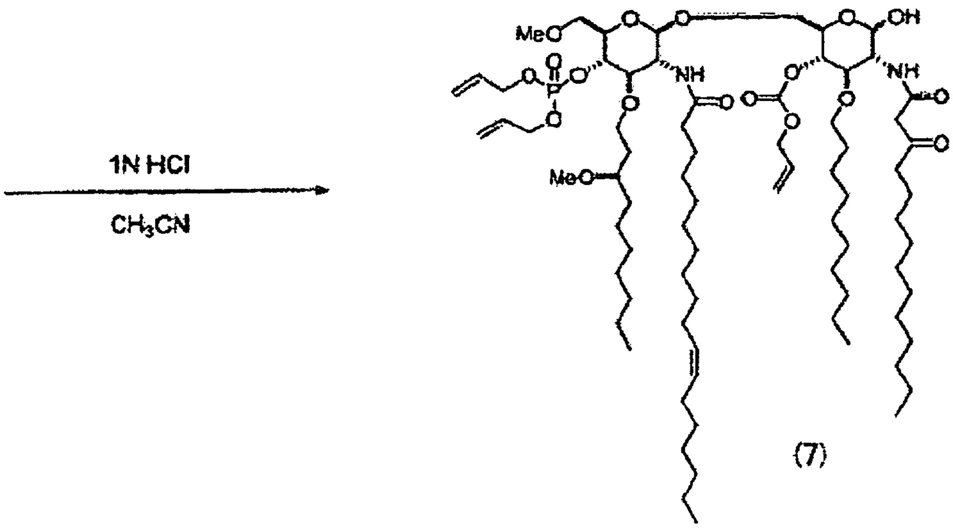


(7)

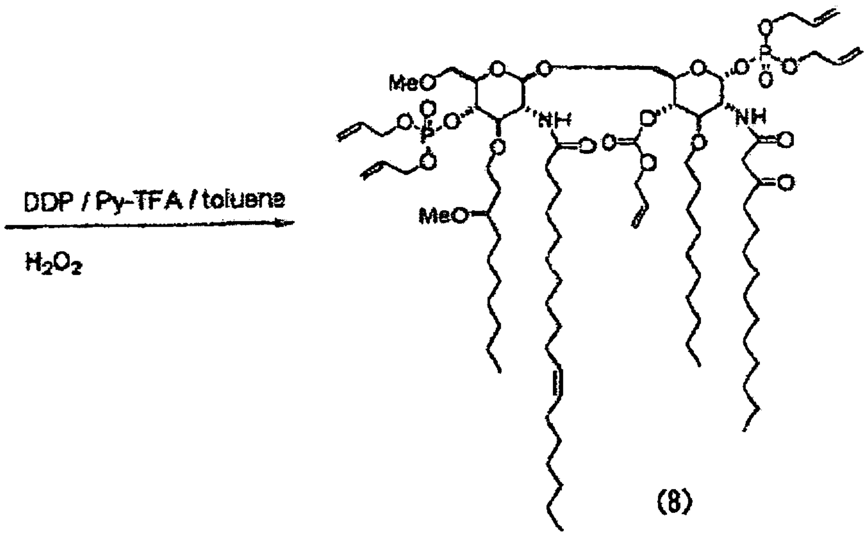


(8)

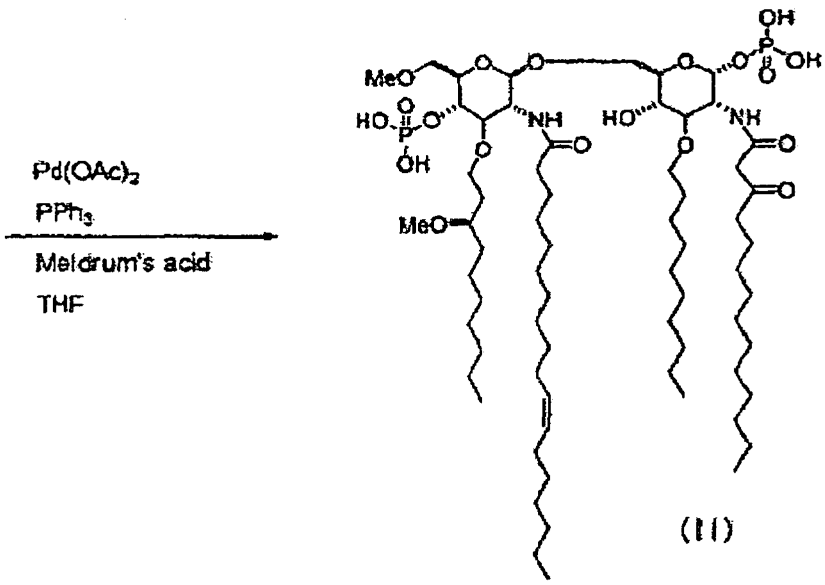


(III)

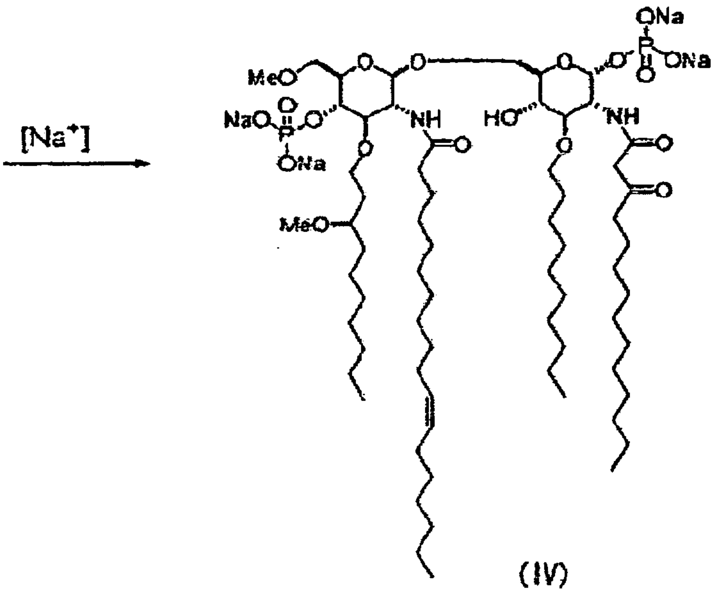


(IV)

The first step of the above production method is a step in which phosphorous acid groups are introduced into the compound of formula (1), after which the compound of formula (2) is produced via an oxidation reaction. Although the solvent used in this step is not particularly limited, an inactive solvent which does not easily react with the starting substances is preferable. For example, ethers such as tetrahydrofuran, diethylether, diisopropylether, dioxane and dimethoxyethane; halogenated hydrocarbons such as chloroform, carbon tetrachloride and 1,2-dichloroethane; hydrocarbons such as hexane and heptane; aromatic hydrocarbons such as benzene and toluene; acetates such as ethyl acetate and methyl acetate; amides such as N,N-dimethylformamide, N-methyl-2-piperidone and hexamethyl phosphorylamide; sulfoxides such as dimethylsulfoxide; and mixed solvents thereof can be cited, among which aromatic hydrocarbon solvents are preferable, and in particular, toluene is optimal.

This process proceeds under relaxed conditions due to the fact that it is performed in the presence of pyridine and trifluoroacetic acid. The pyridine and trifluoroacetic acid in this process can be used in an amount equal to or greater than the amount of the compound of formula (1), but considering the smoothness of the reaction, the refinement process and so forth, 1.0-3.0 equivalents and 1.0-3.0 equivalents, respectively, are preferable, among which 1.0-2.0 equivalents and 1.0-2.0 equivalents, respectively, are optimal.

This process comprises a total of the two steps: introducing the phosphorous acid groups and oxidation. The diallyl diisopropyl phosphoramidate used in the step in which phosphorous acid groups are introduced can be used in an amount equivalent to or greater than the amount of the compound of formula (1), but 1.0-2.0 equivalents is preferable.

The reaction time of the phosphorous acid group introduction step is 0.5-4 hours, preferably 1-2 hours. The reaction temperature is -78°C to room temperature, preferably -40 to 0°C. As the oxidation agent used in the oxidation step, hydrogen peroxide, m-chloroperbenzoic acid, oxone and so forth can be cited, but hydrogen peroxide is optimal. The hydrogen peroxide can be used in an amount equivalent to or greater than the amount of the compound of formula (I), but 1.0-3.0 equivalents is preferable. The reaction time of the oxidation step is 0.5-6 hours, preferably 1-4 hours. The reaction temperature is preferably -50 to 0°C.

The second step of the above production method is a step in which 1-propenyl groups are selectively deprotected by acid hydrolysis from the compound of formula (2), to produce compound (3). Although the solvent used in this step is not particularly limited, an inactive solvent which does not easily react with the starting substances is preferable. For example, alcohols such as methanol, ethanol, isopropanol and tert-butanol; ethers such as tetrahydrofuran, diethylether, diisopropylether, dioxane, dimethoxyethane, diethoxyethane and diglyme; halogenated hydrocarbons such as chloroform, carbon tetrachloride and 1,2-dichloroethane; hydrocarbons such as hexane and heptane; aromatic hydrocarbons such as benzene and toluene; nitriles such as acetonitrile; amides such as N,N-dimethylformamide, N,N-dimethylacetoamide, N-methyl-2-piperidone and hexamethyl phosphorylamide; and sulfoxides such as dimethylsulfoxide can be cited, among which nitriles such as acetonitrile are optimal.

As the acid used in this step, general organic acids and inorganic acids can be cited. As organic acids, monocarboxylic acids such as acetic acid, trifluoroacetic acid, propionic acid and benzoic acid; bicarboxylic acids such as oxalic acid; and organic sulfonic acids such as methane sulfonic acid, tosylic acid and trifluoromethane sulfonic acid can be cited. As inorganic acids, phosphoric acid, hydrochloric acid, sulfuric acid and nitric acid can be used, and inorganic acids such as hydrochloric acid and sulfuric acid are optimal.

The acid used in this step can be used in a catalytic amount or greater with respect to the compound of formula (2), but considering the smoothness of the reaction, the refinement process and so forth, 0.01-1.5 equivalents is preferable, among which 0.1-1.0 equivalents is optimal.

The reaction time is 0.5-12 hours, preferably 1-6 hours. The reaction temperature is 0°C to heating reflux, preferably 10-60°C.

Furthermore, the effect of improving purity is achieved by obtaining the compound of formula (3) as crystals under suitable conditions.

The third step in the above production method is a step in which trichloroacetoimidate groups are introduced as leaving groups into the compound of formula (3) in the presence of a base, to produce the compound of formula (4). The trichloroacetonitrile used in this step can be used in an amount equivalent to or greater than the amount of the compound of formula (3), but considering the smoothness of the reaction, the refinement process and so forth, 1.0-10.0 equivalents is preferable, among which 2.0-5.0 equivalents is optimal.

As the solvent used in this step, ethers such as tetrahydrofuran, diethylether, diisopropylether, dioxane and dimethoxyethane; halogenated hydrocarbons such as chloroform, carbon tetrachloride and 1,2-dichloroethane; acetates such as methyl acetate and ethyl acetate; and mixed solvents thereof with water can be cited, among which a mixed solvent of water and an acetate such as methyl acetate or ethyl acetate is optimal, and the reaction can be performed with good reproducibility.

The mixing ratio of the acetate and water used as the solvent is preferably 1-10% (volume/volume) as the proportion of water, among which 2-5% is optimal.

As the base used in this step, carbonate salts such as sodium carbonate, potassium carbonate and cesium carbonate; hydrogen carbonate salts such as sodium hydrogen carbonate; and alkali metal alkoxides such as sodium methoxide and potassium tert-butoxide can be cited, among which carbonate salts such as potassium carbonate are preferable.

The base used in this step can be used in an amount equal to or greater than the amount of the compound of formula (3), but considering the smoothness of the reaction, the refinement process and so forth, 0.5-3.0 equivalents is preferable, among which 1.0-1.3 equivalents is optimal.

The reaction time is 0.5-24 hours, preferably 1-5 hours. The reaction temperature is preferably -20°C to room temperature, among which -5 to 10°C is optimal.

The fourth step in the above production method is a step in which the compounds of formula (4) and formula (5) are glycyl-bonded, to produce the compound of formula (6). The glycylation reaction can be performed in the presence of an acid catalyst. As the acid catalyst used in this step, organic acids and Lewis acids can be cited. As organic acids, methane sulfonic acid, ethane sulfonic acid, camphorsulfonic acid and p-toluene sulfonic acid are preferable, and in particular, methane sulfonic acid and ethane sulfonic acid are optimal.

As the solvent used in this step, an inactive solvent which does not easily react with the starting substances is preferable. For example, ethers such as tetrahydrofuran, diethylether, diisopropylether, dioxane and dimethoxyethane; halogenated hydrocarbons such as chloroform, carbon tetrachloride and 1,2-dichloroethane; hydrocarbons such as hexane and heptane; aromatic hydrocarbons such as benzene and toluene; and nitriles such as acetonitrile; and mixtures thereof can be cited. Among these, hydrocarbons such as hexane and heptane; aromatic hydrocarbons such as benzene

and toluene; or mixed solvents thereof are preferable, and in particular, a mixed solvent of heptane and toluene is optimal. The reaction temperature is 0°C to heating reflux, preferably 10-30°C. The reaction time is 1 hour to 7 days, preferably 8 hours to 3 days.

The fifth step of the above production method is a step in which 1-propenyl groups are selectively deprotected by acid hydrolysis from the compound of formula (6), to produce compound (7). Although the solvent used in this step is not particularly limited, an inactive solvent which does not easily react with the starting substances is preferable. For example, alcohols such as methanol, ethanol, isopropanol and tert-butanol; ethers such as tetrahydrofuran, diethylether, diisopropylether, dioxane, dimethoxyethane, diethoxyethane and diglyme; halogenated hydrocarbons such as chloroform, carbon tetrachloride and 1,2-dichloroethane; hydrocarbons such as hexane and heptane; aromatic hydrocarbons such as benzene and toluene; nitriles such as acetonitrile; amides such as N-dimethylformamide, N,N-dimethylacetoamide, N-methyl-2-piperidone and hexamethyl phosphorylamide; and sulfoxides such as dimethylsulfoxide can be cited, among which nitriles such as acetonitrile are optimal.

As the acid used in this step, general organic acids and inorganic acids can be cited. As organic acids, monocarboxylic acids such as acetic acid, trifluoroacetic acid, propionic acid and benzoic acid; bicarboxylic acids such as oxalic acid; and organic sulfonic acids such as methane sulfonic acid, tosylic acid and trifluoromethane sulfonic acid can be cited. As inorganic acids, phosphoric acid, hydrochloric acid, sulfuric acid and nitric acid can be used, and inorganic acids such as hydrochloric acid and sulfuric acid are optimal.

The acid used in this step can be used in a catalytic amount or greater with respect to the compound of formula (6), but considering the smoothness of the reaction, the refinement process and so forth, 0.01-1.5 equivalents is preferable, among which 0.1-0.5 equivalents is optimal.

The reaction time is 0.5-12 hours, preferably 1-6 hours. The reaction temperature is 0°C to heating reflux, preferably 10-60°C.

Furthermore, when reaction and processing in this step are performed under reduced pressure, effects such as increased yield, improved operationality and reduced by-products are obtained.

The sixth step of the above production method is a step in which phosphorous acid groups are introduced into the compound of formula (7), after which the compound of formula (8) is produced via an oxidation reaction. Although the solvent used in this step is not particularly limited, an inactive solvent which does not easily react with the starting substances is preferable. For example, ethers such as tetrahydrofuran, diethylether, diisopropylether, dioxane and dimethoxyethane; halogenated hydrocarbons such as chloroform, carbon tetrachloride and 1,2-dichloroethane; hydrocarbons such as hexane and heptane; aromatic hydrocarbons such as benzene and toluene; acetates such as ethyl acetate and methyl acetate; amides such as N,N-dimethylformamide, N-methyl-2-piperidone and hexamethyl phosphorylamide; sulfoxides such as dimethylsulfoxide; and mixed solvents thereof can be cited, among which aromatic hydrocarbon solvents are preferable, and in particular, toluene is optimal.

This process proceeds under relaxed conditions due to the fact that it is performed in the presence of pyridine and trifluoroacetic acid. The pyridine and trifluoroacetic acid used in this process can be used in an amount equal to or greater than the amount of the compound of formula (7), but considering the smoothness of the reaction, the refinement process and so forth, 1.0-3.0 equivalents and 1.0-3.0 equivalents, respectively, are preferable, among which 1.0-2.0 equivalents and 1.0-2.0 equivalents, respectively, are optimal.

This process comprises a total of the two steps: introducing the phosphorous acid groups and oxidation. The diallyl diisopropyl phosphoramidate used in the step in which phosphorous acid groups are introduced can be used in an amount equivalent to or greater than the amount of the compound of formula (7), but 1.5-3.0 equivalents is preferable. The reaction time of the phosphorous acid group introduction step is 0.5-24 hours, preferably 0.5-4 hours. The reaction temperature is -78°C to room temperature, preferably -40 to 0°C. As the oxidation agent used in the oxidation step, hydrogen peroxide, m-chloroperbenzoic acid, oxone and so forth can be cited, but hydrogen peroxide is optimal. The reaction time of the oxidation step is 0.5-6 hours, preferably 1-3 hours. The reaction temperature is preferably -50 to 0°C.

The seventh step in the above production method is a step in which the 2-propenyl groups of the compound of formula (8) are deprotected, to produce the compound of formula (II). Removal of the 2-propenyl groups can be performed by methods stated in the literature- for example, hydrolysis using an acid or a base, or deallylation reaction using a metallic catalyst such as a palladium catalyst. Among these, a deallylation reaction using a metallic catalyst such as a palladium catalyst is preferable, and the use of a zero-valent palladium catalyst such as tetrakis(triphenylphosphine)palladium is particularly preferable. As the zero-valent palladium catalyst such as tetrakis(triphenylphosphine) palladium, a commercially available reagent can be used, but from the viewpoint of reagent stability, producing it within a reaction system is preferable. For example, it is optimal to combine a divalent palladium reagent and a ligand such as triphenylphosphine. As the divalent palladium reagent used in this step, palladium acetate, palladium chloride, bis(triphenylphosphine)palladium (II) chloride and so forth can be cited. For example, when palladium acetate is used as the divalent palladium reagent, the palladium acetate can be used in a catalytic amount with respect to the compound of formula (8), but considering the smoothness of the reaction, the refinement process and so forth, 0.01-0.50 equivalents is preferable, and 0.05-0.25 equivalents is optimal. The amount of triphenylphosphine used can be 1.5-10 equivalents

with respect to the compound of formula (8), but 3.0-5.0 equivalents is preferable. As the nucleophilic agent used in this reaction, a compound which has an active methylene structure within the molecule is preferable. For example, chain-type organic esters such as ethyl cyano acetate; cyclic organic esters such as Meldrum's acid (isopropylidene malonate); and cyclic ketones such as dimedone (5,5-dimethyl-1,3-cyclohexanedione) can be cited, among which cyclic organic esters such as Meldrum's acid and cyclic ketones such as dimedone are optimal from the viewpoint of reducing by-products.

The nucleophilic agent used in this step is used in an amount equal to or greater than the amount of palladium acetate, preferably 10-100 equivalents, more preferably 20-30 equivalents. The reaction time is 1-12 hours, preferably 2-6 hours. The reaction temperature is 10-50°C, preferably 20-40°C.

Although the solvent used in this step is not particularly limited, an inactive solvent which does not easily react with the starting substances is preferable. For example, ethers such as tetrahydrofuran, diethylether, diisopropylether, dioxane and dimethoxyethane; halogenated hydrocarbons such as chloroform, carbon tetrachloride and 1,2-dichloroethane; hydrocarbons such as hexane and heptane; aromatic hydrocarbons such as benzene and toluene; and mixed solvents thereof can be cited, among which tetrahydrofuran in particular is optimal.

The removal method of residual palladium which causes palladium catalysis used in this step is not particularly limited, but sulfur-containing compounds such as trimercaptotriazine and sodium dimethyldithiocarbamate; resin solid adsorbents such as Diaion® CR20; and column chromatography such as a silica gel column chromatography can be used, among which sulfur-containing compounds such as trimercaptotriazine and sodium dimethyldithiocarbamate are optimal.

The eighth step of the above production method is a step in which the compound represented by formula (IV) is produced by adding the required amount of a sodium ion donor source to the compound represented by formula (II).

Although the sodium ion donor source used in this step is not particularly limited, sodium-containing bases such as sodium hydroxide and sodium carbonate can be cited, among which sodium hydroxide is optimal.

Although the solvent used in this step is not particularly limited, an inactive solvent which does not easily react with the starting substances is preferable. For example, alcohols such as methanol, ethanol, isopropanol and tert-butanol; ethers such as tetrahydrofuran, diethylether, diisopropylether, dioxane, and dimethoxyethane; acetates such as ethyl acetate, methyl acetate and isopropyl acetate; ketones such as acetone and methylethylketone; nitriles such as acetonitrile; water; and mixed solvents thereof can be cited, among which alcohols such as methanol, ethanol, isopropanol and tert-butanol are preferable.

The compounds expressed by general formula (II) and the tetrasodium salts represented by general formula (IV) antagonize lipid A, which plays a key role in Gram-negative bacteremia, particularly endotoxin shock, in which there is a high mortality rate caused by endotoxins or lipopolysaccharide (LPS) components present in the outer membranes of Gram-negative bacteria, and they exhibit an excellent anti-endotoxin effect. They also exhibit an antagonistic effect against TLR4, a receptor which recognizes the bacterial body component of bacilli. As a result, they are particularly effective as prevention and treatment agents of sepsis, endotoxemia and improvement of prognosis after coronary artery bypass graft surgery, and they are optimal as active ingredients of medicines.

However, the tetrasodium salts represented by general formula (IV) decompose during storage, albeit slightly, and over time produce trace amounts of impurities. When a sodium salt of the compound represented by general formula (II) is used as a production starting substance of a medicine, it is preferable to suppress decomposition over time as much as possible and suppress generation of impurities.

Thus, in the present invention, as sodium salts of the compound represented by general formula (II), not only can the tetrasodium salts represented by general formula (IV) be used, but sodium salts represented by average formula (I):

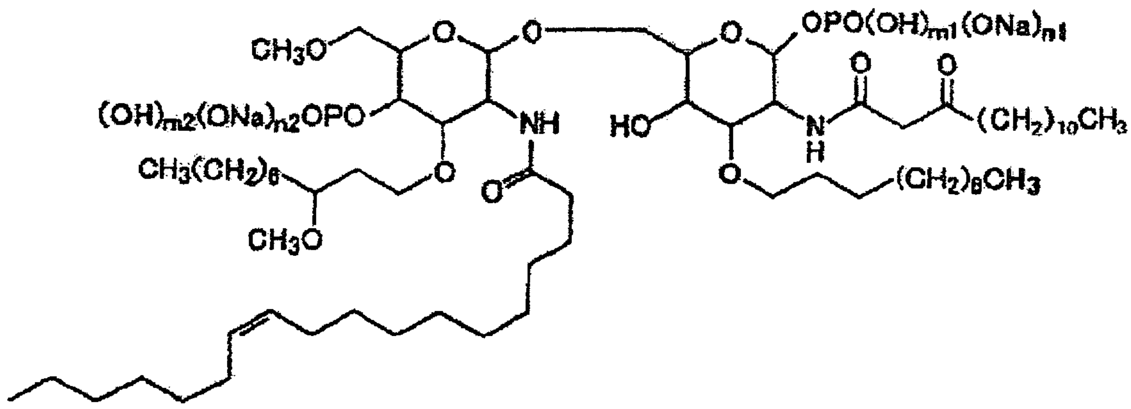

(I)

(in the formula, $m_1$, $n_1$, $m_2$ and $n_2$ independently represent 0 or a positive number not greater than 2, while satisfying $m_1 + n_1 = 2$, $m_2 + n_2 = 2$, $0 < m_1 + m_2 < 4$ and $0 < n_1 + n_2 < 4$) can also be used. The sodium salts represented by average formula (I) can be called sodium acid salts of the compounds represented by general formula (II), because hydrogen atoms are partially present in their phosphoric acid regions. In the sodium salts represented by average formula (I), decomposition over time as well as generation of impurities are suppressed more than in the tetrasodium salts represented by general formula (IV).

In the above average formula (I), it is preferable if $3 \leq n_1 + n_2 < 4$ because fewer impurities are generated, and it is more preferable if $3.5 \leq n_1 + n_2 \leq 3.8$.

Also, the sodium content of the sodium salts represented by the above average formula (I) is preferably at least 5.0 weight% and less than 6.5 weight% because fewer impurities are generated, and it is more preferably at least 5.7 weight% and at most 6.3 weight%.

The sodium salts represented by the above average formula (I) can be prepared by incompletely neutralizing a compound represented by general formula (II) with a sodium-containing base. Here, "incompletely neutralizing" means that the hydrogen atoms in the phosphoric acid region of the compound represented by general formula (II) are not completely consumed (completely neutralized), and neutralization is completed in the state where at least part of the phosphoric acid region of that compound has hydrogen atoms. Therefore, the sodium salts represented by general formula (III):

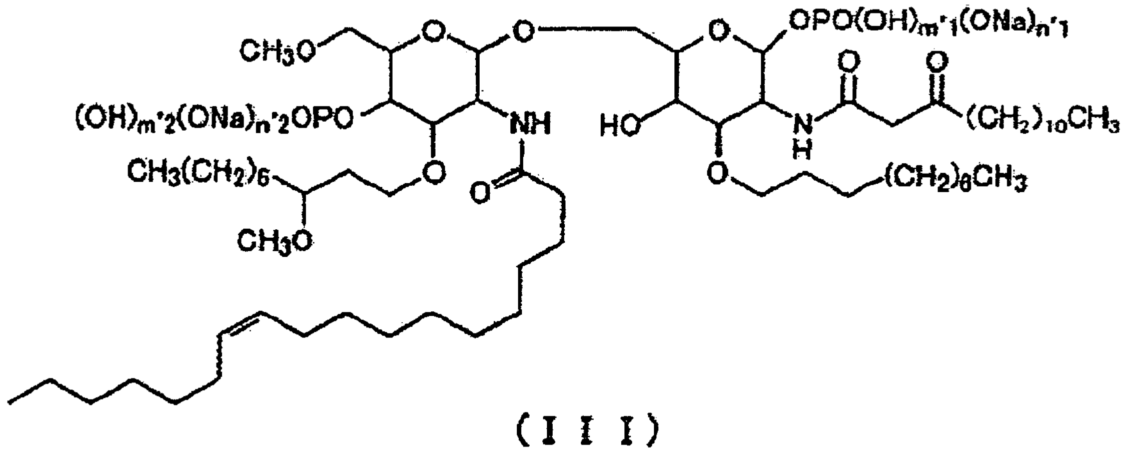

(I I I)

(in the formula, $m_1$, $n_1$, $m_2$ and $n_2$ independently represent 0 or a positive number not greater than 2, while satisfying $m_1 + n_1 = 2$, $m_2 + n_2 = 2$, $0 < m_1 + m_2 < 4$ and $0 < n_1 + n_2 < 4$) are present in the sodium salts represented by the above average formula (1).

Although the sodium-containing base is not particularly limited, sodium hydroxide, sodium carbonate, sodium hydrogen carbonate and so forth can be cited, and sodium hydroxide is optimal. The processing temperature is not particularly limited, and it can be implemented at a temperature of, for example, 10-80°C, preferably 20-40°C.

The aforementioned incomplete neutralization can be implemented in a suitable solvent. The solvent used here

is not particularly limited. For example, alcohols such as methanol, ethanol, isopropanol and tert-butanol; ethers such as tetrahydrofuran, diethylether, diisopropylether, dioxane and dimethoxyethane; acetates such as ethyl acetate, methyl acetate and isopropyl acetate; ketones such as acetone and methylethylketone; nitriles such as acetonitrile; water; and mixed solvents thereof can be used, while alcohols such as methanol, ethanol, isopropanol and tert-butanol are particularly preferable.

The specific method of the aforementioned incomplete neutralization is not particularly limited. For example, a method in which a compound represented by general formula (II) is treated with less than 4 equivalents of a monovalent sodium-containing base such as sodium hydroxide can be cited.

A sodium salt represented by the above average formula (I) is a mixture of various sodium salts. Specifically, a sodium salt represented by average formula (I) is a mixture of a sodium salt represented by general formula (IV) and a sodium salt represented by average formula (III). In other words, a sodium salt represented by average formula (I) corresponds to something in which part of an aggregate made up of only a sodium salt represented by general formula (IV) is substituted with a sodium salt represented by general formula (III).

Therefore, other embodiments of the present invention are a method which suppresses decomposition of the sodium salts represented by general formula (IV) and a method which suppresses generation of impurities in the sodium salts represented by general formula (IV), by making the sodium salts represented by general formula (IV) coexist with the sodium salts represented by general formula (III).

Yet another embodiment of the present invention is a storage method which enables long-term storage by making a sodium salt represented by general formula (IV) coexist with a sodium salt represented by general formula (III). As a result, by the present invention, good storage is possible for at least 1 month, preferably 3 months, more preferably at least 6 months.

Although the implementation temperature of the above decomposition suppression method, the impurity generation suppression method and the storage method are not particularly limited, the implementation temperature is preferably relatively low in order to further suppress decomposition and generation of impurities. For example, it is implemented at 10-80°C, preferably 10-40°C, more preferably 10-25°C.

The sodium salts represented by the above average formula (I) can be administered in an amount which is effective in prevention or treatment of the targeted disease. The dosage can be, for example, 0.01-50 mg, preferably 0.05-25 mg, most preferably 1-12 mg, per person.

The medicine which contains a sodium salt represented by the above average formula (I) can be administered either orally or non-orally, but it is preferably administered non-orally. Non-oral routes include subcutaneous, intravenous, intramuscular and intra-arterial infusion. Here, "infusion" is not limited to injection, but also includes administration via catheter. As a non-oral administration route, intravenous injection is most preferable.

In the case of oral administration, the aforementioned medicine can take the form of granules, powder, tablets, coated tablets, capsules, syrup and so forth. In the case of non-oral administration, it can take the form of an injectable agent (intravenous injectable agent, subcutaneous injectable agent, intramuscular injectable agent and so forth), suppository or external agent (transdermal formulation, ointment and so forth).

In the various formulations above, ordinarily-used excipients, binders, disintegrants, lubricants, dyes, taste and scent agents, and, as necessary, stabilizers, emulsifiers, absorption promoters, surfactants, pH adjusters, preservatives, antioxidants and so forth can be compounded.

## Examples

The present invention is illustrated in further detail by examples below, but the present invention is not limited thereto.

Identification of the compounds pertaining to reference examples 1-7 was performed by comparing the holding time by HPLC, with the compounds synthesized by the production method disclosed in WO2004/074303 as a reference. Quantification of the compounds was calculated from intensity obtained from a UV detector by HPLC from the standard curve created with the compounds synthesized by the production method disclosed in WO2004/074303 as a reference.

Although the solid phase that can be used in HPLC is not particularly limited, a reverse phase column such as C18 (ODS), C4, C8, C22 or C30 is preferable. Although the mobile phase is not particularly limited, a solvent such as acetonitrile, methanol or water, or a mixed solvent thereof, is preferable, and if desired, good peak separation is obtained by adding an acid such as perchloric acid, trifluoroacetic acid, acetic acid or phosphoric acid, or a salt thereof, or an amine such as triethylamine or diethylamine. Additionally, reproducibility of peak separation and holding time is improved by holding the column temperature constant by a column oven and so forth.

[Reference example 1]

Synthesis of α-D-glucopyranose, (1Z)-1-propenyl 2-deoxy-3-O-[(3R)-3-methoxydecyl]-6-O-methyl-2-{[(11Z)-1-oxo-11-octadecenyl]amino}-, 4-(di-2-propenyl phosphate)

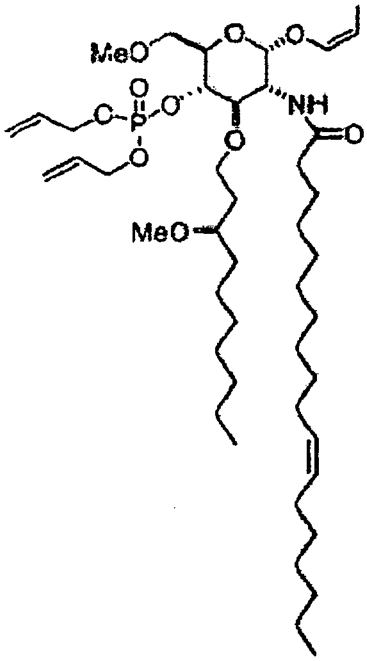

In a 2-liter 4-necked flask, 235 g of α-D-glucose, (1Z)-1-propenyl 2-deoxy-3-O-[(3R)-3-methoxydecyl]-6-O-methyl-2-{[(11Z)-1-oxo-11-octadecenyl]-amino}- (CAS registration no.: 748165-17-5) was dissolved in 933 ml of toluene, after which 129 ml of diallyl N,N-diisopropyl phosphoramidate, 39.4 ml of pyridine and 36.3 ml of trifluoroacetic acid were dripped in in that order at room temperature. 1.5 hours after dripping was finished, the reaction solution was cooled to -20°C, and acetonitrile dilute solution (933 ml) containing 47.5 ml of hydrogen peroxide was dripped in over the course of 37 minutes. After dripping was finished, it was heated to 10°C over the course of 40 minutes. After 3 hours, the reaction was quenched by adding 940 ml of 5% sodium hydrogen sulfite solution, and this was heated to room temperature. The solution which was extracted with ethyl acetate and stored in a refrigerator was used in the next reaction as is, as a solution of the titled compound.

[Reference example 2]

Synthesis of α-D-glucose, 2-deoxy-3-O-[(3R)-3-methoxydecyl]-6-O-methyl-2-{[(11Z)-1-oxo-11-octadecenyl]amino}-, 4-(di-2-propenyl phosphate)

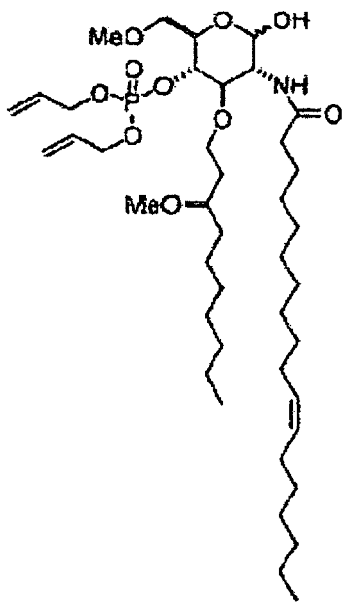

The α-D-glucopyranose, (1Z)-1-propenyl 2-deoxy-3-O-[(3R)-3-methoxydecyl]-6-O-methyl-2-{[(11Z)-1-oxo-11-octadecenyl]amino}-, 4-(di-2-propenyl phosphate) solution obtained in reference example 1 was washed with 699 ml of 1 N hydrochloric acid aqueous solution, then 27.9 ml of 5N hydrochloric acid aqueous solution was added, and this was stirred for 5 hours at room temperature. After being neutralized with 699 ml of 5% sodium bicarbonate aqueous solution, it was separated with ethyl acetate, and the organic layer was washed with 699 ml of 5% sodium chloride aqueous solution. This was dried by adding 69.9 g of anhydrous magnesium sulfate, and then filtered, and the filtrate was concentrated under reduced pressure. 466 ml of acetone was added to the residue, and this was again concentrated under reduced pressure. This acetone treatment was repeated, and 289.1 g (content ratio: 92.1%, contained quantity: 266.3 g) of crude substance of the titled compound was obtained. Yield: 97%.

1065 ml of acetonitrile was added to 289.1 g of the obtained crude substance, and this was stirred for 5 minutes at 20°C, after which it was cooled to 0°C for 4 hours, and stirred for another 4 hours. The precipitated crystals were filtered out, and then dried overnight under reduced pressure at room temperature, and the equivalent of 228.6 g of the titled compound was obtained.

[Reference example 3]

Synthesis of α-D-glucopyranose, 2-deoxy-3-O-[(3R)-3-methoxydecyl]-6-O-methyl-2-{[(11Z)-1-oxo-11-octadecenyl]amino}-, 4-(di-2-propenyl phosphate) 1-(2,2,2-trichloroethane imidate)

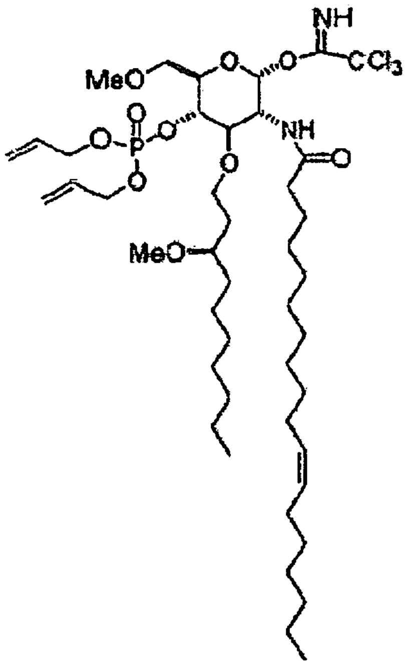

In a 2-liter 4-necked flask, 280 g of α-D-glucose, 2-deoxy-3-O-[(3R)-3-methoxydecyl]-6-O-methyl-2-{[(11Z)-1-oxo-11-octadecenyl]-amino}-, 4-(di-2-propenyl phosphate), 46.8 g of potassium carbonate, 560 ml of methyl acetate, 170 ml of trifluoroacetonitrile and 8.4 ml of water were added, and this was stirred for 2 hours in a nitrogen atmosphere at 0°C. The reaction solution was filtered with celite, and concentrated under reduced pressure at 40°C. Then, azeotropy was performed 3 times with 560 ml of heptane, and 432 g (content: 63.9%, containing 171.4 ml of heptane) of the titled compound was obtained. Yield: 87.5%.

[Reference example 4]

Synthesis of α-D-glucopyranoside, (1Z)-1-propenyl 6-O-{4-O-[bis(2-propenyloxy)phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 4-(2-propenyl carbonate)

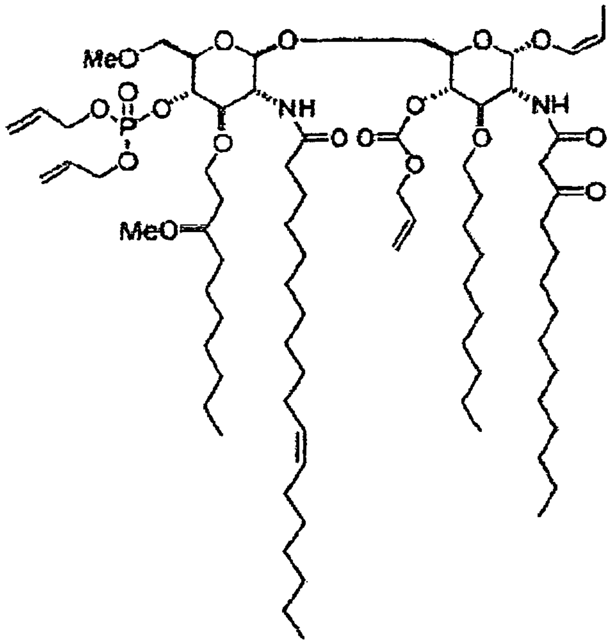

In a 2-liter 4-necked flask, a heptane solution (content: 50.4%) of 410.8 g of α-D-glucopyranose, 2-deoxy-3-O-[(3R)-3-methoxydecyl]-6-O-methyl-2-{[(11Z)-1-oxo-11-octadecenyl]amino}-, 4-(di-2-propenyl phosphate) 1-(2,2,2-trichloroethane imidate), 249.7 ml of heptane, 105.9 g of α-D-glucopyranoside, (1Z)-1-propenyl 3-O-decyl-2-deoxy-2-[1,3-dioxotetradecyl]amino]-, 4-(2-propenyl carbonate) (CAS registration no.: 185955-29-7), 140 ml of toluene and 2.89 ml of methanesulfonic acid were added in that order, and this was stirred for 15 hours in a nitrogen atmosphere at 25°C. Then, 2000 ml of ethyl acetate and 1000 ml of water were added to the reaction solution, and after extraction and separation, the organic layer was washed with 1000 ml of 5% sodium hydrogen carbonate aqueous solution and 1000 ml of 10% sodium chloride aqueous solution in that order. After concentration under reduced pressure (warm bath 45-50°C), 800 ml of methanol was added to the residue, this was concentrated, the same operations were repeated, and a crude substance of the titled compound was obtained.

1920 ml of methanol was added to the obtained crude substance, and the impurities were filtered with celite. The impurities and celite were washed with methanol. In addition, 1400 ml of methanol as added to the solution, after which it was cooled to 17°C, and 375 ml of water was dripped in. After that, it was cooled to -20°C and stirred for 45 minutes, and then filtered. The filtrate was washed with 400 ml of 90% water-containing methanol which had been cooled beforehand to 0°C, then this was dried as is under reduced pressure using a nutsche, and 427.2 g of moist substance was obtained.

427.2 g of the moist substance was put in a 10-liter 4-necked flask, and 2400 ml of methanol was added and it was dissolved. After cooling to 10°C, 180 ml of water was dripped in. After dripping was finished, it was cooled to 0°C, and stirred for 50 minutes, after which it was filtered. The filtrate was washed with 400 ml of 90% water-containing methanol which had been cooled beforehand to 0°C, then this was dried under reduced pressure at 35°C, and 199.5 g (content: 92.2%) of the titled compound was obtained. Yield: 92.6%.

[Reference example 5]

Synthesis of α-D-glucopyranose, 6-O-{4-O-[bis(2-propenyloxy)phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 4-(2-propenyl carbonate)

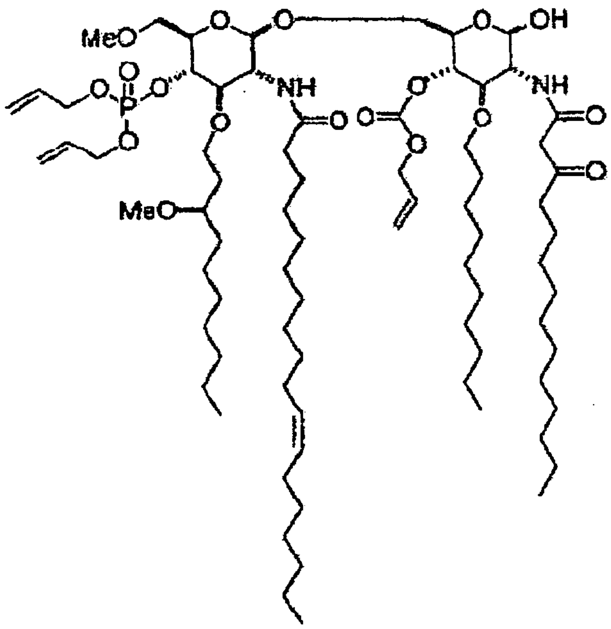

In a 10-liter 4-necked flask, 199.0 g (content: 92.2%) of α-D-glucopyranoside, (1Z)-1-propenyl 6-O-{4-O-[bis(2-propenyloxy)phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 4-(2-propenyl carbonate), 1990 ml of acetonitrile and 34.6 ml of 1N hydrochloric acid aqueous solution were added, and this was stirred for 2 hours at 30°C at 130 hPa of vacuum. Additionally, the degree of vacuum and temperature were gradually raised, and finally, the acetonitrile was concentrated to 3/4 volume-wise at 106 hPa of vacuum. 995 ml of 10% sodium chloride aqueous solution and 1493 ml of ethyl acetate were added to the concentrate, and extraction was performed. After that, the organic layer was washed with 95 ml of 5% sodium hydrogen carbonate aqueous solution and 995 ml of 10% sodium chloride aqueous solution in that order. The organic layer was dried with 60 g of anhydrous magnesium sulfate, and then filtered. The filtrate was concentrated, and 640 ml of toluene was added to the residue, and it was dissolved, and 778.1 g (content: 155.6 g equivalent) of toluene solution of the titled compound was obtained. Yield: 87.2%.

[Reference example 6]

Synthesis of α-D-glucopyranose, 6-O-{4-O-[bis(2-propenyloxy)phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 1-(di-2-propenyl phosphate) 4-(2-propenyl carbonate)

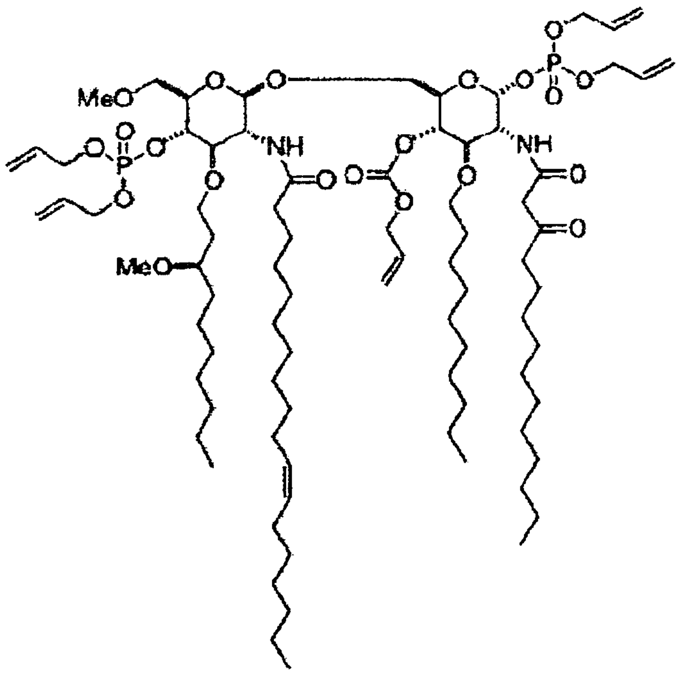

550.6 g (content: 110 g equivalent) of toluene solution of α-D-glucopyranose, 6-O-{4-O-[bis(2-propenyloxy) phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 4-(2-propenyl carbonate) was concentrated under reduced pressure at 50°C. 440 ml of toluene was added to the residue, it was dissolved, and the solution was concentrated under reduced pressure at bath temperature 45-50°C. Another 440 ml of toluene was put in, then nitrogen replacement was performed, and 537.6 g (content: 109.13 g) of toluene solution was produced. This solution was concentrated under reduced pressure and then dried, and 665 ml of toluene was added, and nitrogen replacement was performed. 11.91 ml of trifluoroacetic acid was added and this was stirred for 15 hours, after which 12.50 ml of pyridine was added. This was cooled to -20°C, after which 37.15 ml of diallyl N,N-diisopropyl phosphoramidate was dripped in. 30 minutes after dripping was finished, it was cooled to -30°C, and 15.17 ml of 30% hydrogen peroxide was dripped in. 6 minutes after dripping was finished, the isothermal tank was set to -20°C. After 1 hour 10 minutes, the reaction was quenched by adding 655 ml of 5% sodium thiosulfate aqueous solution. 655 ml of ethyl acetate was added, extraction was performed. The organic layer was washed with 655 ml of 0.5N hydrochloric acid aqueous solution, 655 ml of 10% sodium chloride aqueous solution, 655 ml of 5% sodium bicarbonate aqueous solution and 655 ml of 10% sodium chloride aqueous solution, in that order. This was dried by adding 43.7 g of anhydrous magnesium sulfate, after which filtration was performed. The filtrate was concentrated under reduced pressure, and 159.0 g of the titled compound (content: 101.6 g) was obtained. Yield: 83.5%.

[Reference example 7]

Synthesis of α-D-glucopyranose, 3-O-decyl-2-deoxy-6-O-(2-deoxy-3-O-[(3R)-3-methoxydecyl]-6-O-methyl-2-{[(11Z)-1-oxo-11-octadecenyl]amino}-4-O-phosphono-β-D-glucopyranosyl}-2-[(1,3-dioxotetradecyl)amino]-, 1-(dihydrogen phosphate)

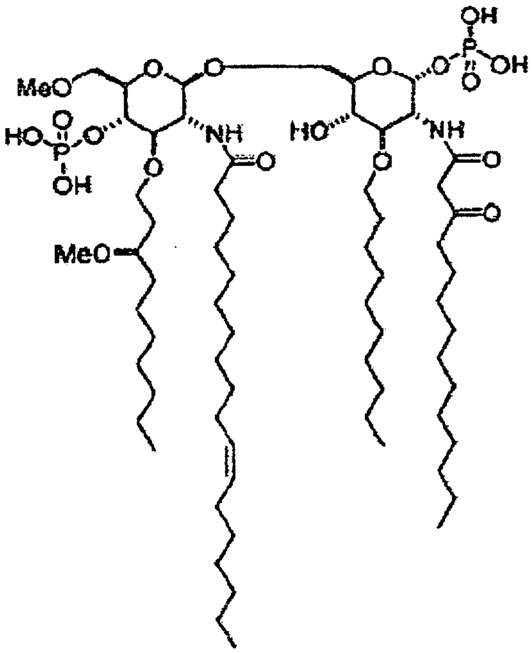

70.49 g of Meldrum's acid, 2.93 g of palladium acetate and 51.3 g of triphenylphosphine were put in a 3-liter 4-necked flask. After nitrogen replacement, 1321 ml of tetrahydrofuran was added, and a tetrahydrofuran solution (203 ml) of 101.6 g of α-D-glucopyranose, 6-O-{4-O-[bis(2-propenyloxy)phosphinyl]-2-deoxy-3-O-[(3R)-3-methoxydecyl]-6-O-methyl-2-{[(11Z)-1-oxo-11-octadecenyl]amino}-β-D-glucopyranosyl}-3-O-decyl-2-deoxy-2-[(1,3-dioxotetradecyl)amino]-, 1-(di-2-propenyl phosphate) 4-(2-propenyl carbonate) was added, and this was stirred for 2 hours at 32°C, after which it was stirred for an additional 4 hours at 30°C. Then, 250 ml of methanol was added to the reaction solution, this was concentrated under reduced pressure, and 466.7 g of residue was obtained. To this, 4570 ml of methanol was added, this was heated to 40°C and dissolved, after which 5.55 g of trimercaptotriazine was added, and this was stirred overnight at room temperature. The precipitated trimercaptotriazine-palladium complex was filter-separated, and then washed with methanol, and 4330 g of filtrate was obtained.

3908.2 ml of this methanol solution was concentrated under reduced pressure, and 440.9 g of residue was obtained. 450 ml of acetone was added to the residue, it was concentrated under reduced pressure, and then 450 ml of acetone was again added and it was concentrated. After the residue was stored in a refrigerator overnight, 1800 ml of acetone was added, it was heated to 40°C, and stirred for 1.5 hours. It was stirred for 1.5 hours while air cooling at 30°C or less, after which it was filtered. The filtrate was washed with 750 ml of acetone, after which the removed solids were dried under reduced pressure at 35-40°C, and 104.48 g (content: 74.2%) of free acid of the titled compound was quantitatively obtained as a crude substance.

[Column refinement]

1) Hydrophobic adsorption resin column refinement

93.5 g (free acid content: 64.5%) of the aforementioned free acid was dissolved in 10 liters of methanol, after which 1110 ml of purified water was added, and some of the unnecessary matter was polish-filtered. The filtrate was washed with 50 ml of 90% methanol/water, and the washing solution and filtrate were combined. This solution was loaded into a hydrophobic adsorption resin column (load ratio: 1% w/column volume, SEPABEADS® SP20SS, Mitsubishi Chemical Corp.). It was eluted with 90% methanol/water, and the free acid was refined. Yield was 54.3 g as free acid (by HPLC quantification) (column yield: 90%).

2) Anion exchange resin column refinement

Half of the main fraction of the hydrophobic adsorption resin column (SEPABEADS® SP20SS) was loaded into an anion exchange resin column (load ratio: 0.5% w/column capacity, TOYOPEARL® DEAE650M, Tosoh Corp.). After loading was finished, the free acid was refined by passing 95% methanol/water (mobile phase A) and 95% methanol/water (containing 0.25 mol/l sodium acetate) (mobile phase B) through an anion exchange resin column (TOYOPEARL® DEAE650M) under gradient conditions. The same operation was performed for the other half as well, and a total of 48.3 g (by HPLC quantification) as free acid was obtained (column yield: 89%).

[Sedimentation of refined substance]

17.6 liters of acetonitrile at 25°C was dripped into 11 liters (48.3 g as free acid) of the main fraction in the aforementioned anion exchange resin column refinement. After dripping was finished, stirring was continued for 2 hours, and the precipitated sediment was filtered under reduced pressure. When the filtered-out sediment was dried under reduced pressure (bath temperature 25°C), the yield of sediment was 46.9 g. When the obtained sediment underwent HPLC quantification analysis and the sodium content measurement described below, free acid content was 89.5% (w/w), 42.0 g (yield: 87%), and Na content was 4.80% (w/w) (calculated as anhydride).

[Example 1]

39.0 g (free acid content: 34.5 g) of the main fraction sediment of the DEAE column and 942.8 liters [sic] of methanol were put in a 3-liter 4-necked flask, and stirred at 25°C. 127.3 ml of 0.2N sodium hydroxide-methanol solution was added to the 3-liter 4-necked flask, after which it was stirred overnight. The solution was polish-filtered, and the filtrate was moved to a 10-liter 4-necked flask. 3413 ml of acetone at 25°C was dripped into the 10-liter 4-necked flask to which the filtrate had been moved. The precipitated sediment was filtered out and dried under reduced pressure at 25°C, and a salt of the aforementioned sodium salt was obtained. Yield was 37.0 g, and the content of free acid salt was 89.46% (w/w), 33.1 g (yield: 95.9%).

[Sodium content measurement]

The sodium content of the salt obtained in example 1 was measured. 25 ±2.5 mg of sample (sediment) was precisely weighed out and put into a 50-ml conical tube. 5 ml of methanol was added, and it was completely dissolved. To the obtained solution, 20 ml of 2.5 mmol/liter oxalic acid aqueous solution was added, and this was homogenized by stirring, making a sample solution (n = 2). Meanwhile, the same operation was performed to prepare a blank solution ($BL_{smp.}$) as a reference for the sample solution (n = 1).

To 3 ml of sodium ion standard solution (1000 mg/liter), a mixed solution of 2.5 mmol/liter oxalic acid aqueous solution/methanol (80:20, v/v) was added to make 50 ml, and this was used as the sodium standard solution (n = 1). Meanwhile, the same operation was performed to prepare a blank solution ($BL_{std.}$) for the sodium standard solution (n = 1).

Using an ion chromatography (IC) system equipped with an electrical conductivity detector, an analysis column (a tube of inside diameter 4.6 mm and length 10 cm packed with silica get of diameter approx. 7 μm to which carboxyl groups were bonded as cation exchange groups; for example, SHIM-PACK IC-C3 made by Shimadzu Corp.), a guard column 1 (a tube of inside diameter 6.0 mm and length 5 cm packed with triacontyl silylated silica gel of diameter approx. 20 μm; for example, ERP20 made by Develosil) and a guard column 2 (a tube of inside diameter 4.6 mm and length 7.5 mm packed with the same particles as the analysis column; for example, SHIM-PACK IC-GC3 made by Shimadzu Corp.), the column temperature was set to 40°C, and 10 μl each of sample solution, sodium standard solution and blank solution were poured in at a flow rate of 1.0 ml/minute (mobile phase: 2.5 mmol/oxalic acid aqueous solution), the ion peak area was determined, and the sodium content was determined by the formula below.

$$\text{Sodium content (\%) (as is)} = \frac{(A_{smp.} - A_{BLsmp.})}{(A_{std.} - A_{BLstd.})} \times \frac{f}{W_{smp.}} \times \frac{3}{20}$$

$$\text{Sodium ion content (\%) (calculated as anhydride)} = \text{Sodium ion content (\%) (as is)} \times \frac{100}{(100 - KF_{smp.})}$$

| | |
|---|---|
| $A_{smp.}$: | Sodium ion peak area in sample solution (average of n = 2) |
| $ABL_{smp.}$: | Sodium ion peak area in blank solution ($BL_{smp}$) with respect to sample solution (average of n = 2) |
| $A_{std}$.: | Sodium ion peak area in sodium standard solution (average of n = 2) |
| $ABL_{std.}$: | Sodium ion peak area in blank solution ($BL_{std.}$) with respect to sodium standard solution (average of n = 2) |
| f: | Result of indication of reference value shown in sodium ion standard solution (expressed as mg/liter) |
| $W_{smp.}$: | Weighed amount of sample (mg) |

$KF_{smp.}$: Moisture content of sample (%)

As a result, the sodium content of the salt obtained in example 1 was 6.09 weight%, and since the molecular weight of the free acid was 1313.68, the atomic weight of sodium is 23.00, and the atomic weight of hydrogen is 1.01, then 1313.68 × 6.09/(2300 - 21.99 × 6.09) = 3.70, and therefore it was determined to be 3.70 Na salt ($n_1 + n_2 = 3.70$ in average formula (I)).

[Example 2]

By performing the operations up to sedimentation of the aforementioned refined substance, a salt having sodium content of 4.75 weight% (calculated as anhydride) (2.84 Na salt) was obtained.

[Example 3]

Using 6.0 g of DEAE main fraction sediment, 74.5 ml of methanol for dissolution, 15.2 ml of 0.2N sodium hydroxide-methanol solution and 270 ml of acetone, salt having sodium content of 6.05 weight% (calculated as anhydride) (3.67 Na salt) was obtained by the same operations as in the aforementioned example 1.

[Example 4]

Using 1.84 g of DEAE main fraction sediment, 21.4 ml of methanol for dissolution, 6.3 ml of 0.2N sodium hydroxide-methanol solution and 83 ml of acetone, salt having sodium content of 6.45 weight% (calculated as anhydride) (3.93 Na salt) was obtained by the same operations as in the aforementioned example 1.

[Comparative example 1]

Using 2.47 g of DEAE main fraction sediment, 35.4 ml of methanol for dissolution, 8.7 ml of 0.2N sodium hydroxide-methanol solution and 111 ml of acetone, salt having sodium content of 6.65 weight% (calculated as anhydride) (4.06 Na salt) was obtained by the same operations as in the aforementioned example 1.

[Storage safety test]

The salts of examples 2-4 and comparative example 1 were put in glass bottles with screw caps, and stored for 30 days at 25°C, and the generation ratio of impurities A, B and C were determined as follows.

Each salt of examples 2-4 and comparative example 1 was dissolved in methanol to make a solution of concentration 5 mg/ml. When HPLC analysis was performed under the conditions shown in Table 1 for each solution and blank solution (methanol only), three types of decomposition product were identified, and the generation ratio (converted to a monthly basis) was calculated for each of the three decomposition products (impurities A, B and C) as a percentage of area.

Table 1

| | | | |
|---|---|---|---|
| Column | YMC Carotenoid, C-30, 250 x 4.6 mm, 5 μm | | |
| Column temperature | 35°C | | |
| Flow rate | 1.0 ml/minute | | |
| Gradient | Time | Solution A (%) | Solution B (%) |
| | 0 minutes | 100 | 0 |
| | 15 minutes | 100 | 0 |
| | 35 minutes | 0 | 100 |
| | 40 minutes | 0 | 100 |
| Injected quantity | 25 μl | | |

(continued)

| Gradient | Time | Solution A (%) | Solution B (%) |
|---|---|---|---|
| Detection | UV 254 nm and 208 nm | | |
| Solution A: Obtained by mixing 980 ml of methanol and 20 ml of phosphoric acid, and degassing<br>Solution B: Obtained by mixing 500 ml of methanol and 500 ml of dichloromethane, taking 980 ml of it and fully mixing with 20 ml of phosphoric acid, and then degassing | | | |

Results are shown in Tables 2-5.

Table 2

| Impurity A<br>Days | 2.84 Na salt | 3.67 Na salt | 3.93 Na salt | 4.06 Na salt |
|---|---|---|---|---|
| 0 | 0.00 | 0.00 | 0.00 | 0.00 |
| 1.0 | - | 0.00 | 0.03 | 0.06 |
| 1.9 | - | 0.01 | 0.04 | 0.08 |
| 4.5 | - | 0.01 | 0.06 | 0.12 |
| 18 | 0.00 | - | - | - |
| Generation ratio (%/month) | 0.00 | 0.072 | 0.363 | 0.729 |

Table 3

| Impurity B<br>Days | 2.84 Na salt | 3.67 Na salt | 3.93 Na salt | 4.06 Na salt |
|---|---|---|---|---|
| 0 | 0.00 | 0.08 | 0.14 | 0.18 |
| 1.0 | - | 0.12 | 0.26 | 0.36 |
| 1.9 | - | 0.09 | 0.29 | 0.48 |
| 4.5 | - | 0.13 | 0.38 | 0.67 |
| 18 | 0.00 | - | - | - |
| Generation ratio (%/month) | 0.00 | 0.267 | 1.455 | 3.117 |

Table 4

| Impurity C<br>Days | 2.84 Na salt | 3.67 Na salt | 3.93 Na salt | 4.06 Na salt |
|---|---|---|---|---|
| 0 | 0.00 | 0.00 | 0.00 | 0.00 |
| 1.0 | - | 0.00 | 0.00 | 0.00 |
| 1.9 | - | 0.00 | 0.00 | 0.00 |
| 4.5 | - | 0.01 | 0.01 | 0.00 |
| 8.8 | - | 0.01 | 0.01 | 0.00 |
| 18 | 0.17 | - | - | - |

(continued)

| Impurity C / Days | 2.84 Na salt | 3.67 Na salt | 3.93 Na salt | 4.06 Na salt |
|---|---|---|---|---|
| 29.5 | - | 0.07 | 0.06 | 0.03 |
| Generation ratio (%/month) | 0.282 | 0.072 | 0.063 | 0.033 |

Table 5

| Impurity generation ratio | | | Impurity A | Impurity B | Impurity C |
|---|---|---|---|---|---|
| | Ex. 2 | 2.84 Na salt (Na content: 4.75 wt%) | 0.000 | 0.000 | 0.282 |
| | Ex. 3 | 3.67 Na salt (Na content: 6.05 wt%) | 0.072 | 0.267 | 0.072 |
| | Ex. 4 | 3.93 Na salt (Na content: 6.45 wt%) | 0.363 | 1.455 | 0.063 |
| | Comp. ex. 1 | 4.06 Na salt (Na content: 6.65 wt%) | 0.729 | 3.117 | 0.033 |

The results of Table 5 are shown in FIG. 1 and FIG. 2. In FIG. 1, the horizontal axis corresponds to the value of $n_1 + n_2$ of the salts of examples 2-4 and comparative example 1, and in FIG. 2, the horizontal axis corresponds to the sodium content of the salts of examples 2-4 and comparative example 1. From FIG. 1 it is seen that the impurity generation ratios of examples 2-4, in which $n_1 + n_2 < 4$, are lower than that of comparative example 1, in which $n_1 + n_2 = 4$. The impurity generation ratio is lower in the range of $3 \leq n_1 + n_2 < 4$, particularly in the range of $3.5 \leq n_1 + n_2 \leq 3.8$. Similarly, from FIG. 2 it is seen that the impurity generation ratios of examples 2-4, in which sodium content is less than 6.65 weight%, are lower than that of comparative example 1, in which it is 6.65 weight%. The impurity generation ratio is lower in the range of at least 5.0 weight% and less than 6.5 weight%, particularly in the range of at least 5.7 weight% and at most 6.3 weight%.

Industrial Applicability

The salts represented by average formula (I) of the present invention are useful as prevention or treatment agents of sepsis, endotoxemia and improvement of prognosis after coronary artery bypass graft surgery, and because they have particularly excellent stability over time, they can be used as active ingredients of formulations which can be stored for a long time.

Additionally, the salts represented by general formula (III) of the present invention can improve long-term stability of the salts represented by general formula (IV) which are similarly effective as prevention or treatment agents of sepsis, endotoxemia and improvement of prognosis after coronary artery bypass graft surgery, and therefore, they can be used as manufacturing starting materials of medicines which can be stored for a long time by being used together with the salts represented by general formula (IV).

## Claims

**1.** A sodium salt represented by average formula (I):

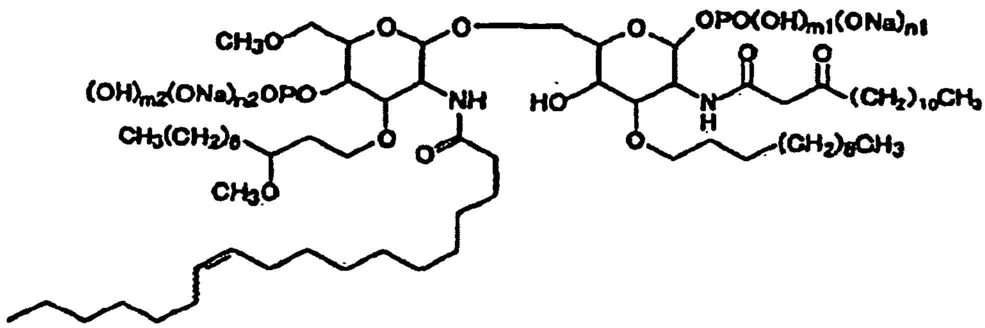

(I)

wherein $m_1$, $n_1$, $m_2$ and $n_2$ independently represent 0 or a positive number not greater than 2, while satisfying
$m_1 + n_1 = 2$,
$m_2 + n_2 = 2$,
$0 < m_1 + m_2 < 4$ and
$3.5 \leq n_1 + n_2 \leq 3.8$.

**2.** The sodium salt according to claim 1, wherein the sodium salt is a mixture of a sodium salt represented by general formula (III):

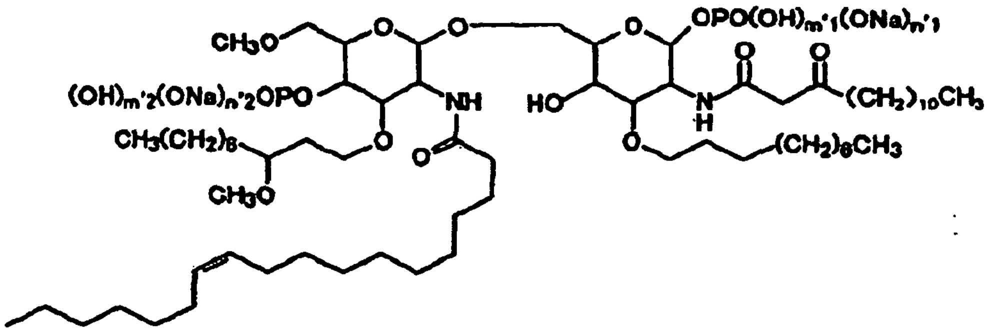

(III)

wherein, $m'_1$, $n'_1$, $m'_2$ and $n'_2$ independently represent 0 or an integer not greater than 2, while satisfying $m'_1 + n'_1 = 2$, $m'_2 + n'_2 = 2$, $0 < m'_1 + m'_2 < 4$ and $0 < n'_1 + n'_2 < 4$, and a sodium salt represented by general formula (IV):

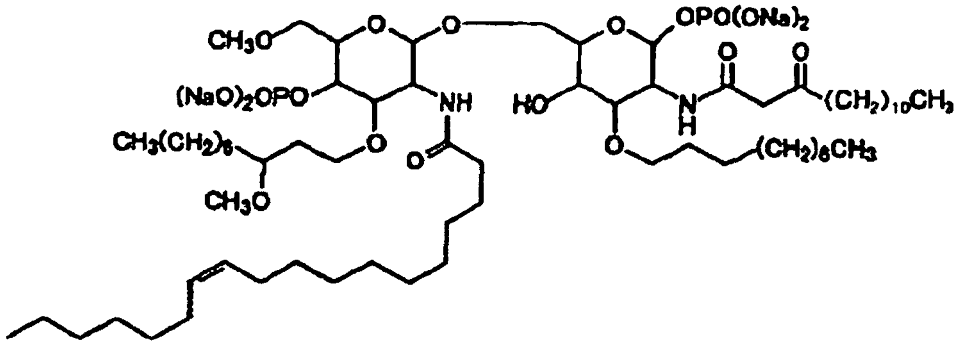

(IV).

**3.** The sodium salt according to claim 1, wherein the sodium salt is obtainable by incomplete neutralization of a compound represented by general formula (II):

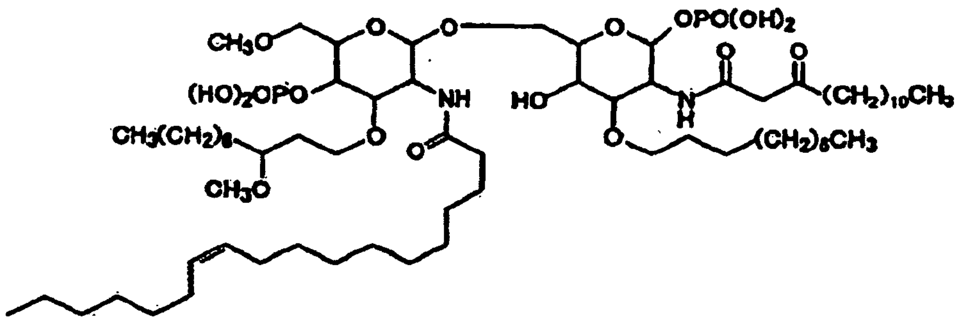

(II)

with a sodium-containing base.

4. The sodium salt according to claim 1, wherein sodium content is at least 5.7 weight % and at most 6.3 weight %.

5. A method of producing the sodium salt according to any of claims 1, 3, and 4, **characterized in that** a compound represented by general formula (II):

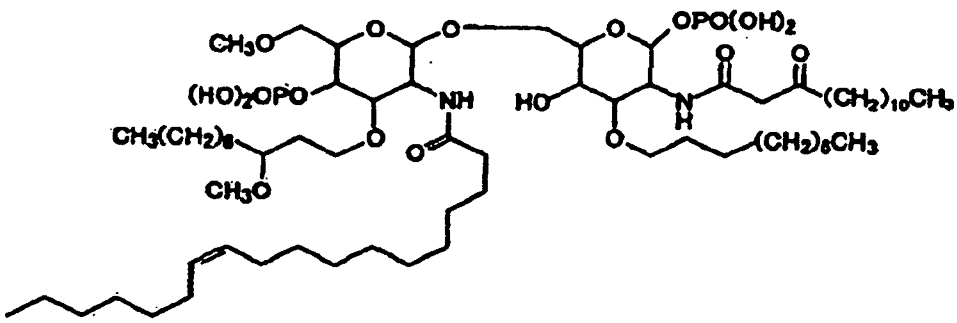

(II)

is incompletely neutralized by a sodium-containing base.

6. A formulation comprising the sodium salt according to any one of claims 1 to 4.

7. A decomposition suppression method of a sodium salt represented by general formula (IV):

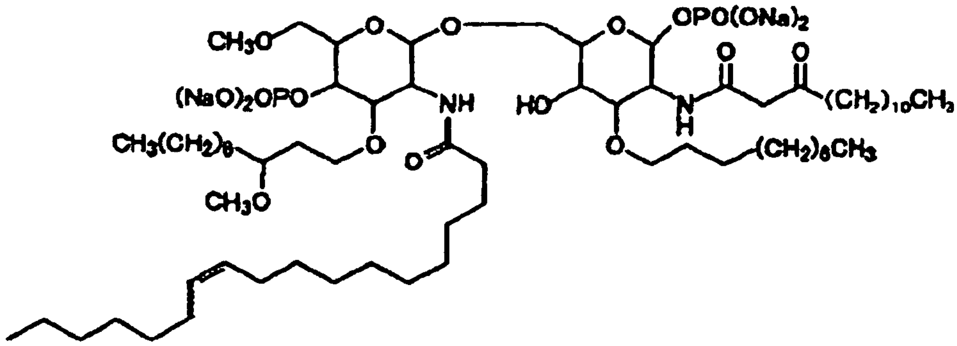

(IV)

**characterized in that** a sodium salt of general formula (IV) is made to coexist with a sodium salt represented by general formula (III):

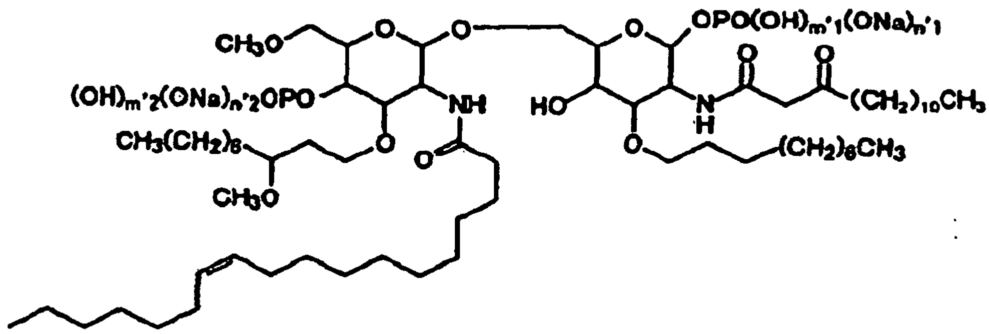

(III)

wherein, $m'_1$, $n'_1$, $m'_2$ and $n'_2$ independently represent 0 or an integer not greater than 2, while satisfying $m'_1$,+ $n'_1$=2, $m'_2 + n'_2 = 2$,$0 < m'_1 + m'_2 < 4$ and $0 < n'_1 + n'_2 < 4$ to yield the sodium salt according to claim 1.

**8.** An impurity generation suppression method in a sodium salt represented by general formula (IV):

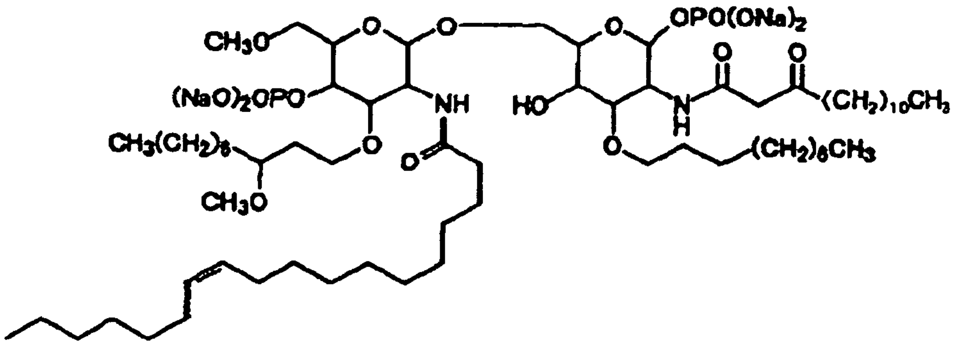

(IV)

**characterized in that** a sodium salt of general formula (IV) is made to coexist with a sodium salt represented by general formula (III):

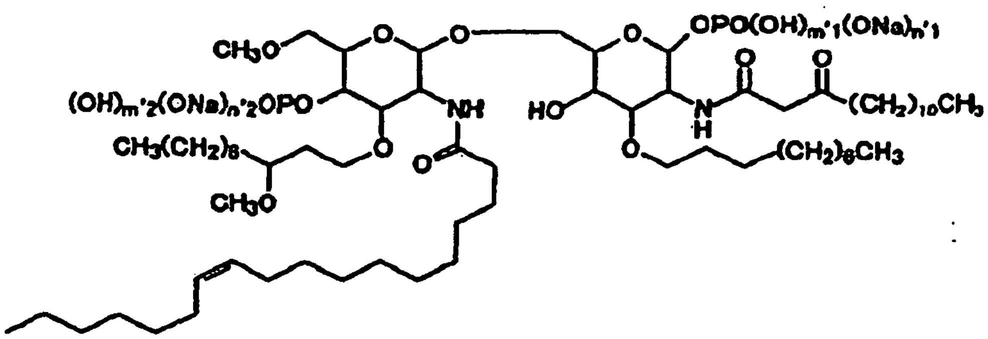

(III)

wherein, $m'_1$, $n'_1$, $m'_2$ and $n'_2$ independently represent 0 or an integer not greater than 2, while satisfying $m'_1 + n'_1 = 2$, $m'_2 + n'_2 = 2$, $0 < m'_1 + m'_2 < 4$ and $0 < n'_1 + n'_2 < 4$ to yield the sodium salt according to claim 1.

**9.** A storage method of a sodium salt represented by general formula (IV):

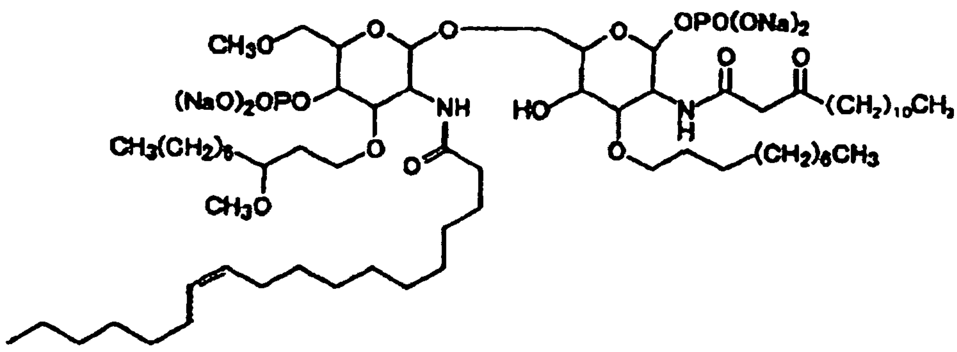

(IV)

**characterized in that** a sodium salt of general formula (IV) is made to coexist with a sodium salt represented by general formula (III):

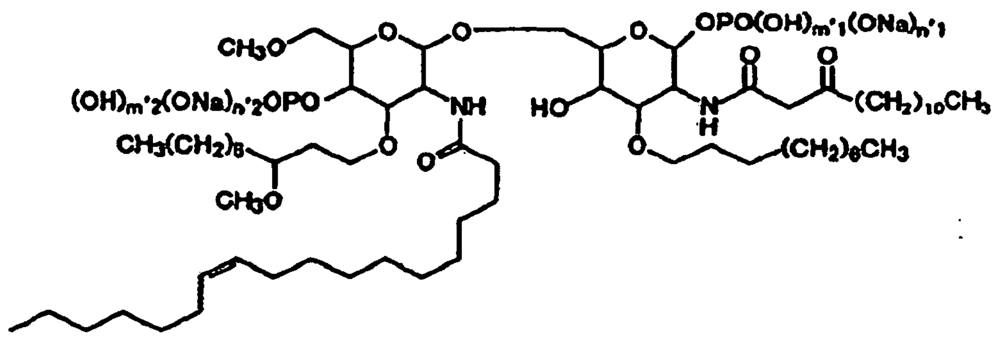

(III)

wherein, $m'_1$, $n'_1$, $m'_2$ and $n'_2$ independently represent 0 or an integer not greater than 2, while satisfying $m'_1 + n'_1 = 2$, $m'_2 + n'_2 = 2,0 < m'_1 + m'_2 < 4$ and $0 < n'_1 + n'_2 < 4$ to yield the sodium salt according to claim1.

**10.** A formulation according to claim 6, wherein the formulation exhibits less decomposition as compared to a formulation comprising the compound represented by general formula (IV) in the absence of the compound represented by general formula (III).

**Patentansprüche**

**1.** Natriumsalz, dargestellt durch die gemittelte Formel (I) :

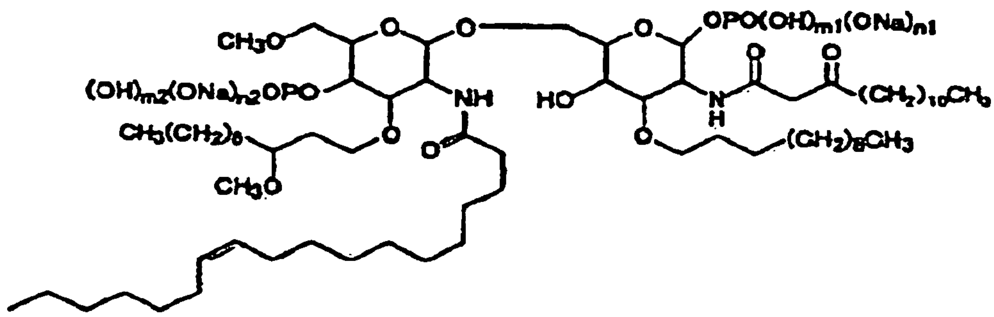

(I)

worin $m_1$, $n_1$, $m_2$ und $n_2$ unabhängig voneinander 0 oder eine positive Zahl, die nicht größer als 2 ist, darstellen, und folgendes erfüllen:
$m_1 + n_1 = 2$
$m_2 + n_2 = 2$,
$0 < m_1 + m_2 < 4$ und
$3{,}5 < n_1 + n_2 \leq 3{,}8$.

**2.** Natriumsalz gemäß Anspruch 1, worin das Natriumsalz eine Mischung eines Natriumsalzes der allgemeinen Formel (III):

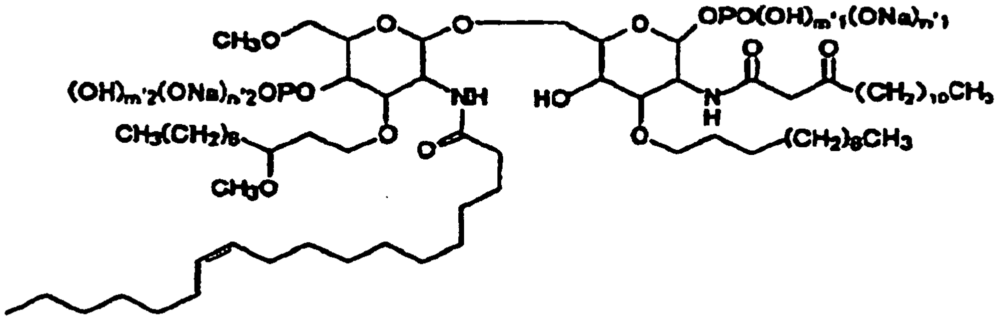

(III)

worin $m'_1$, $n'_1$, $m'_2$ und $n'_2$ unabhängig voneinander 0 oder eine ganze Zahl, die nicht größer als 2 ist, darstellen, und folgendes erfüllen: $m'_1 + n'_1 = 2$, $m'_2 + n'_2 = 2$, $0 < m'_1 + m'_2 < 4$ und $0 < n'_1 + n'_2 < 4$ und einem Natriumsalz der allgemeinen Formel (IV) ist:

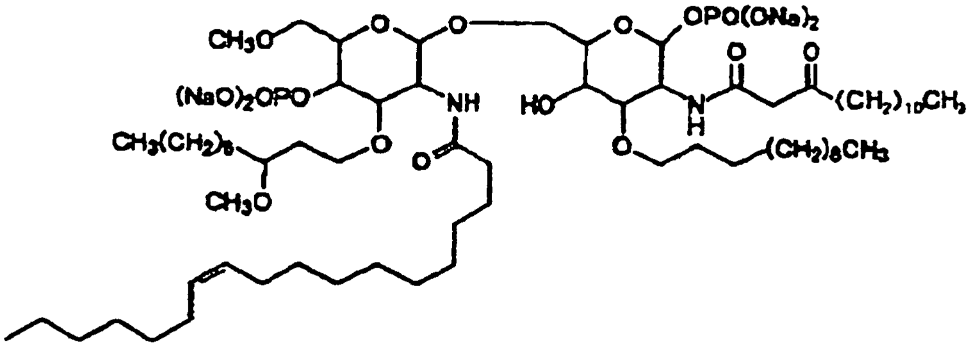

(IV).

**3.** Natriumsalz gemäß Anspruch 1, worin das Natriumsalz durch unvollständige Neutralisierung einer Verbindung der allgemeinen Formel (II)

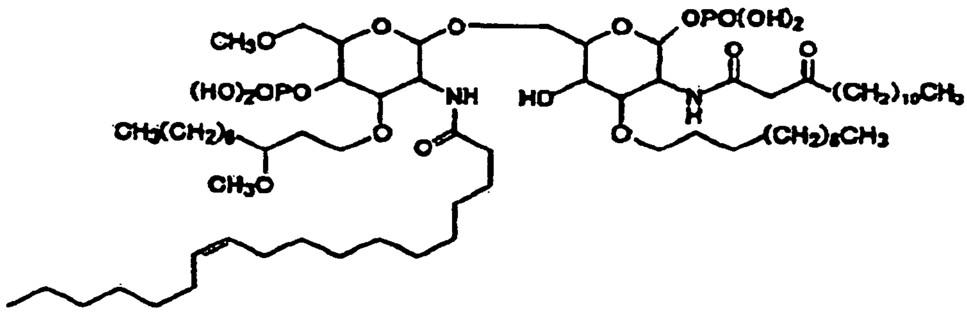

(II)

mit einer Natrium enthaltenden Base erhältlich ist.

4. Natriumsalz gemäß Anspruch 1, worin der Natriumgehalt mindestens 5,7 Gew.% und höchstens 6,3 Gew.% ist.

5. Verfahren zur Herstellung eines Natriumsalzes gemäß irgendeinem der Ansprüche 1, 3 und 4, **dadurch gekennzeichnet, dass** eine Verbindung der allgemeinen Formel (II)

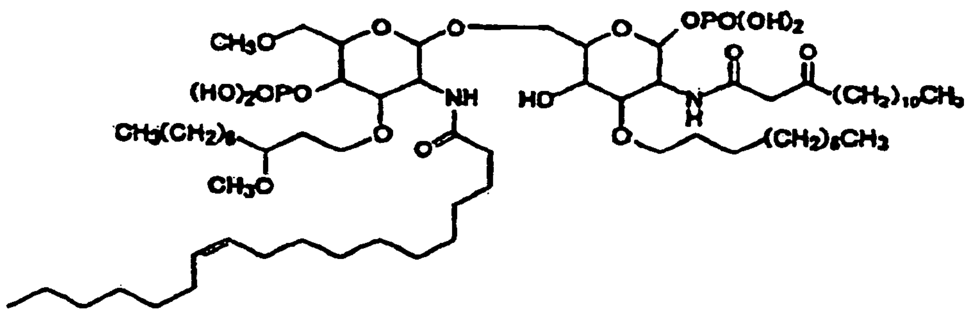

(II)

unvollständig mit einer Natrium enthaltenden Base neutralisiert wird.

6. Zubereitung, die das Natriumsalz gemäß irgendeinem der Ansprüche 1 bis 4 umfasst.

7. Verfahren zur Unterdrückung der Zersetzung eines Natriumsalzes der allgemeinen Formel (IV)

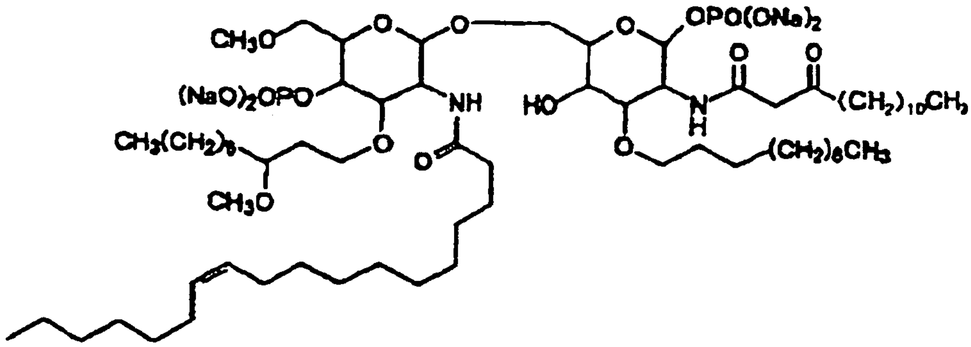

(IV).

**dadurch gekennzeichnet, dass** ein Natriumsalz der allgemeinen Formel (IV) dazu gebracht wird, mit einem Natriumsalz der allgemeinen Formel (III) zu koexistieren:

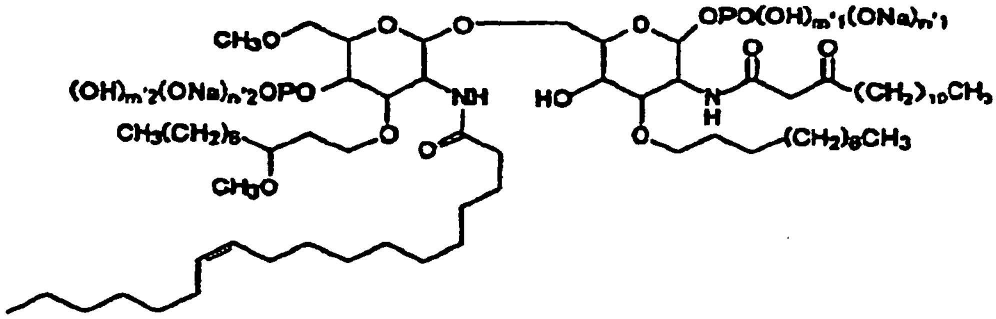

(III)

worin $m'_1$, $n'_1$, $m'_2$ und $n'_2$ unabhängig voneinander 0 oder eine ganze Zahl, die nicht größer als 2 ist, darstellen, und folgendes erfüllen, $m'_1 + n'_1 = 2$, $m'_2 + n'_2 = 2$, $0 < m'_1 + m'_2 < 4$ und $0 < n'_1 + n'_2 < 4$, um das Natriumsalz gemäß Anspruch 1 zu ergeben.

**8.** Verfahren zum Unterdrücken von Verunreinigungsbildung in einem Natriumsalz der Formel (IV)

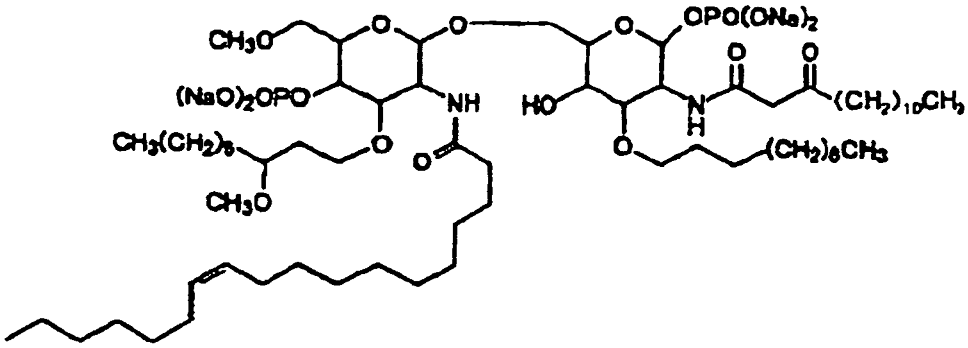

(IV).

**dadurch gekennzeichnet, dass** ein Natriumsalz der allgemeinen Formel (IV) dazu gebracht wird, mit einem Natriumsalz der allgemeinen Formel (III) zu koexistieren:

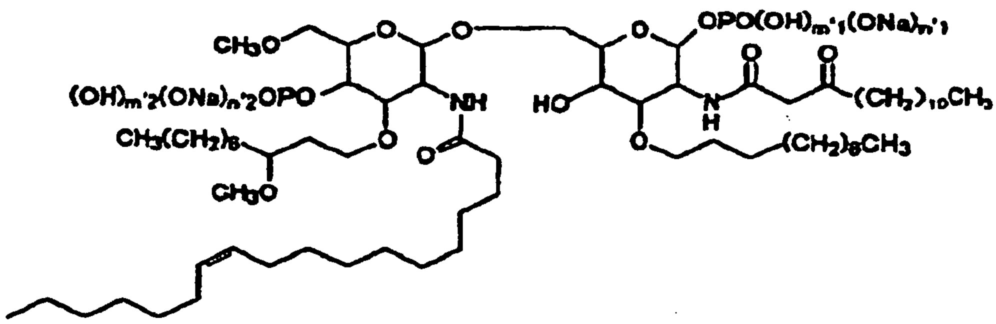

(III)

worin $m'_1$, $n'_1$, $m'_2$ und $n'_2$ unabhängig voneinander 0 oder eine ganze Zahl, die nicht größer als 2 ist, darstellen, und folgendes erfüllen, $m'_1 + n'_1 = 2$, $m'_2 + n'_2 = 2$, $0 < m'_1 + m'_2 < 4$ und $0 < n'_1 + n'_2 < 4$, um das Natriumsalz gemäß Anspruch 1 zu ergeben.

**9.** Lagerverfahren für ein Natriumsalz der allgemeinen Formel (IV)

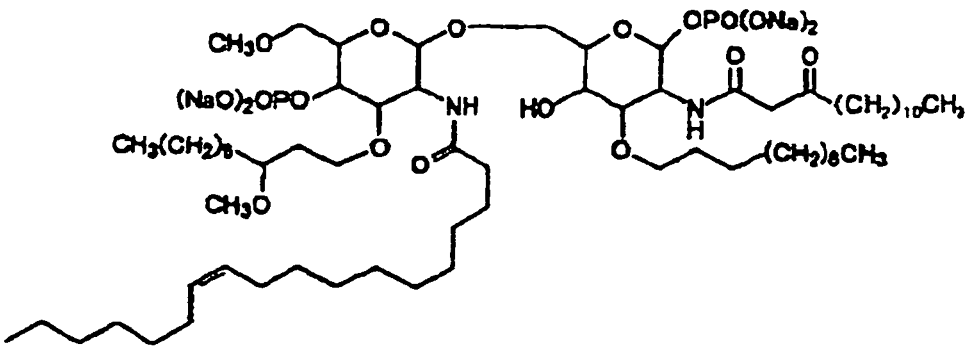

(IV).

**dadurch gekennzeichnet, dass** ein Natriumsalz der allgemeinen Formel (IV) dazu gebracht wird, mit einem Natriumsalz der allgemeinen Formel (III) zu koexistieren:

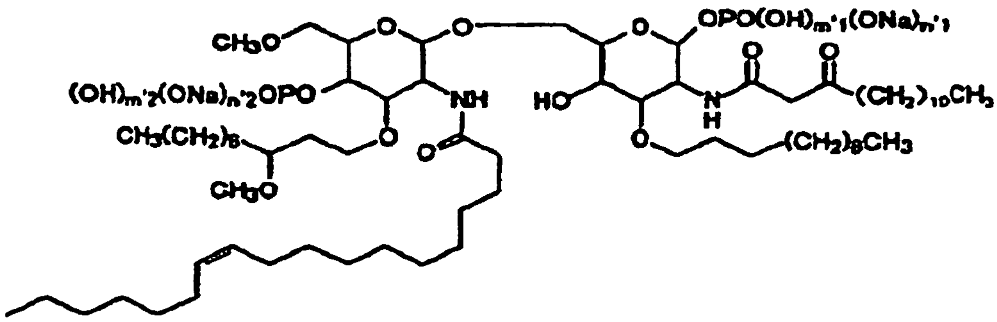

(III)

worin $m'_1$, $n'_1$, $m'_2$ und $n'_2$ unabhängig voneinander 0 oder eine ganze Zahl, die nicht größer als 2 ist, darstellen, und folgendes erfüllen, $m'_1 + n'_1 = 2$, $m'_2 + n'_2 = 2$, $0 < m'_1 + m'_2 < 4$ und $0 < n'_1 + n'_2 < 4$, um das Natriumsalz gemäß Anspruch 1 zu ergeben.

**10.** Zubereitung gemäß Anspruch 6, worin die Zubereitung im Vergleich zu einer Zubereitung, die die Verbindung der allgemeinen Formel (IV) in Abwesenheit der Verbindung der allgemeinen Formel (III) umfasst, weniger Zersetzung zeigt.

## Revendications

**1.** Sel de sodium représenté par la formule moyenne (I) :

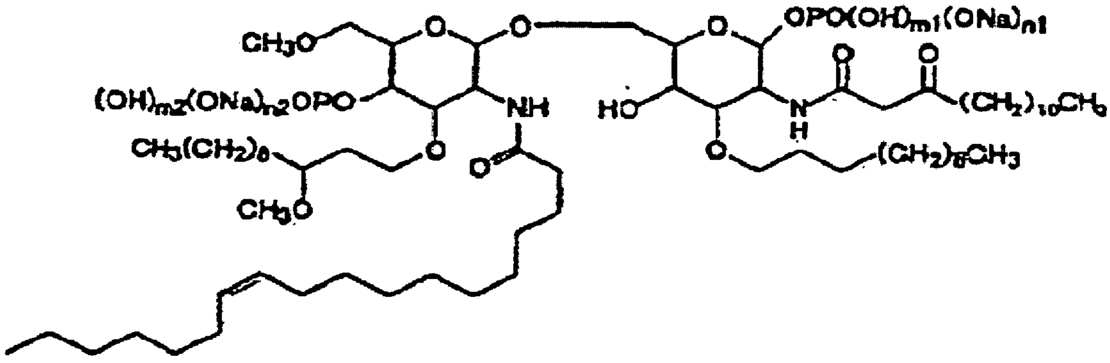

(I)

dans laquelle $m_1$, $n_1$, $m_2$ et $n_2$ représentent indépendamment 0 ou un nombre positif non supérieur à 2, tout en satisfaisant à
$m_1 + n_1 = 2$,
$m_2 + n_2 = 2$,
$0 < m_1 + m_2 < 4$ et
$3,5 \leq n_1 + n_2 \leq 3,8$.

**2.** Sel de sodium selon la revendication 1, dans lequel le sel de sodium est un mélange d'un sel de sodium représenté par la formule générale (III) :

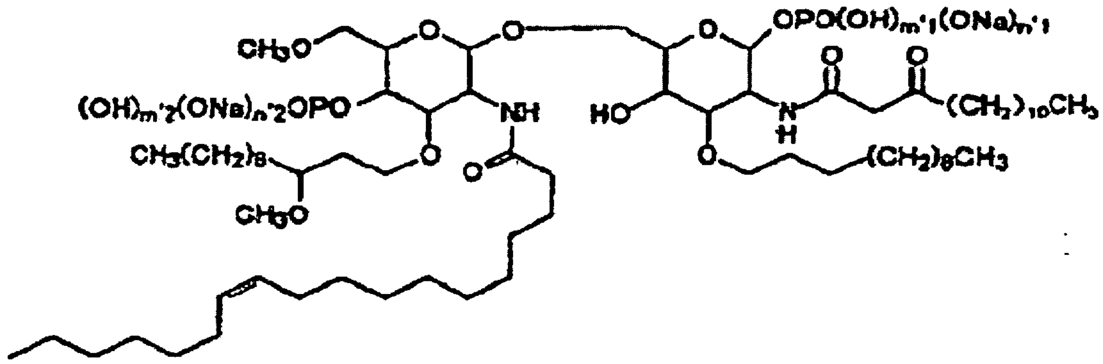

(III)

dans laquelle $m'_1$, $n'_1$, $m'_2$ et $n'_2$ représentent indépendamment 0 ou un entier non supérieur à 2, tout en satisfaisant à $m'_1 + n'_1 = 2$, $m'_2 + n'_2 = 2$, $0 < m'_1 + m'_2 < 4$ et $0 < n'_1 + n'_2 < 4$, et d'un sel de sodium représenté par la formule générale (IV) :

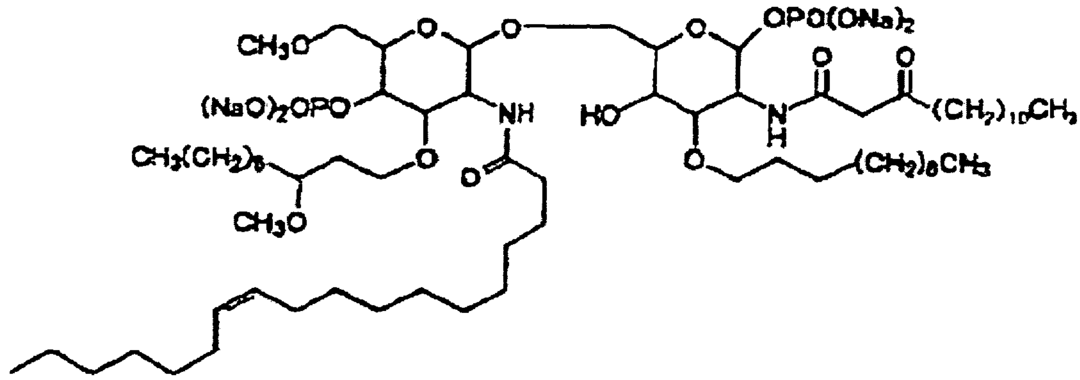

(IV).

3. Sel de sodium selon la revendication 1, dans lequel le sel de sodium peut être obtenu par neutralisation incomplète d'un composé représenté par la formule générale (II) :

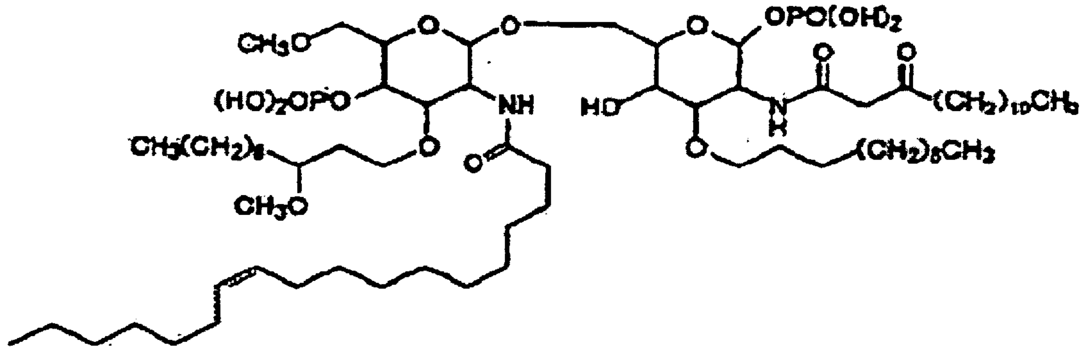

(II)

avec une base contenant du sodium.

4. Sel de sodium selon la revendication 1, dans lequel la teneur en sodium est au moins 5,7% en poids et au plus 6,3% en poids.

5. Procédé de production du sel de sodium selon l'une quelconque des revendications 1, 3 et 4, **caractérisé en ce qu'**un composé représenté par la formule générale (II) :

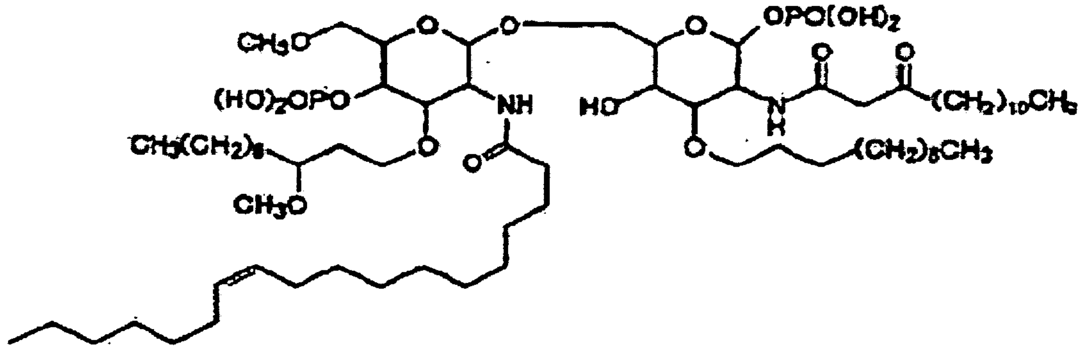

(II)

est incomplètement neutralisé par une base contenant du sodium.

**6.** Formulation contenant le sel de sodium selon l'une quelconque des revendications 1 à 4.

**7.** Procédé d'élimination de décomposition d'un sel de sodium représenté par la formule générale (IV) :

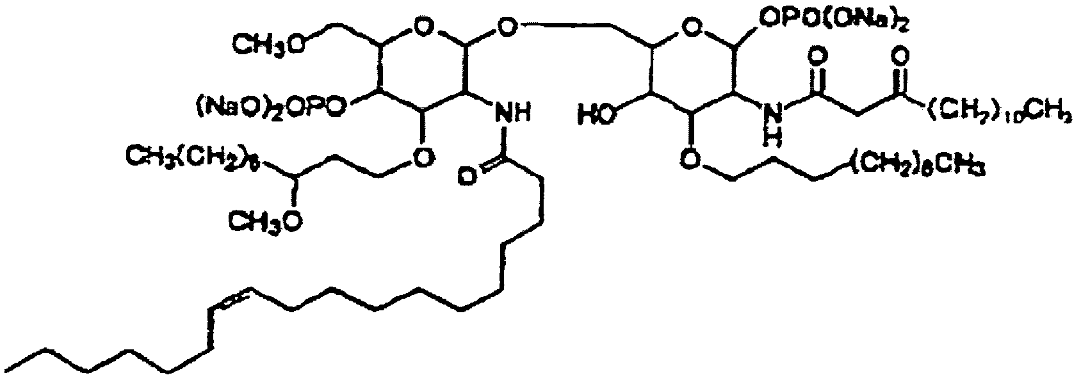

(IV).

**caractérisé en ce qu'**un sel de sodium de formule générale (IV) est fait pour coexister avec un sel de sodium représenté par la formule.générale (III) :

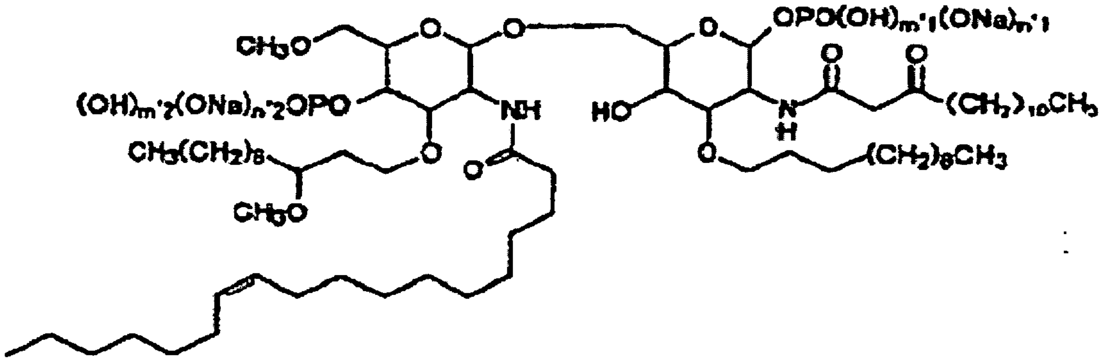

(III)

dans laquelle $m'_1$, $n'_1$, $m'_2$ et $n'_2$ représentent indépendamment 0 ou un entier non supérieur à 2, tout en satisfaisant à $m'_1 + n'_1 = 2$, $m'_2 + n'_2 - 2$, $0 < m'_1 + m'_2 < 4$ et $0 < n'_1 + n'_2 < 4$ pour donner le sel de sodium selon la revendication 1.

**8.** Procédé d'élimination de la génération d'impuretés dans un sel de sodium représenté par la formule générale (IV) :

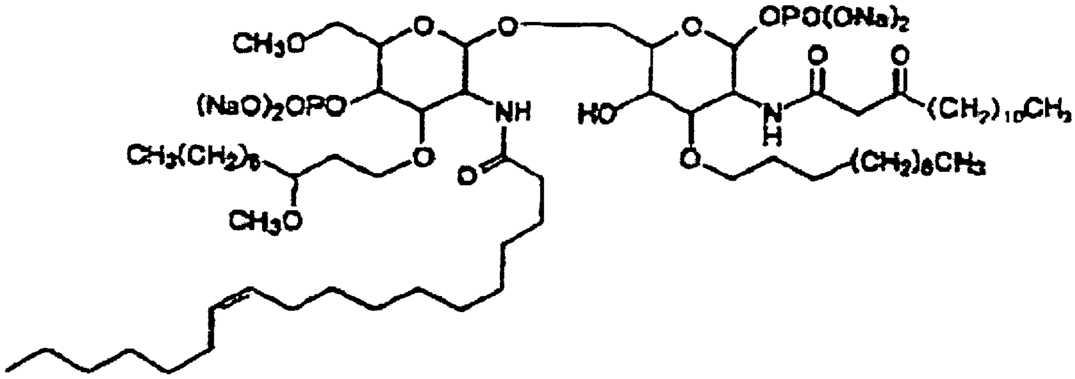

(IV).

**caractérisé en ce qu'**un sel de sodium de formule générale (IV) est fait pour coexister avec un sel de sodium représenté par la formule générale (III) :

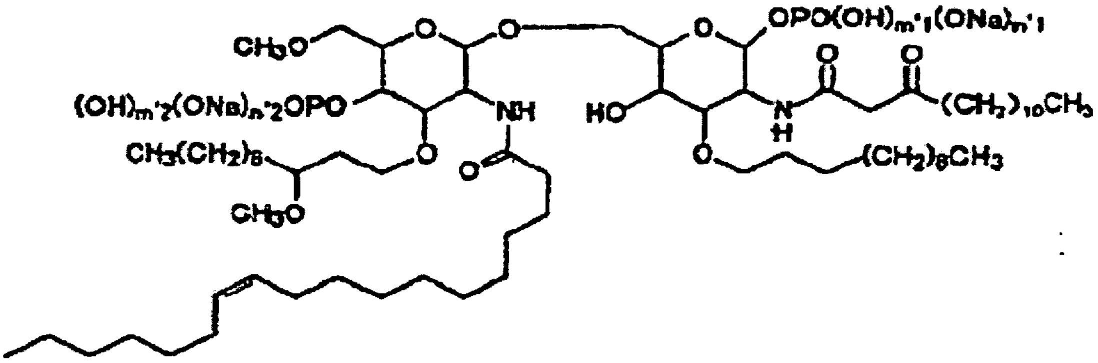

(III)

dans laquelle $m'_1$, $n'_1$, $m'_2$ et $n'_2$ représentent indépendamment 0 ou un entier non supérieur à 2, tout en satisfaisant à $m'_1 + n'_1 = 2$, $m'_2 + n'_2 = 2$, $0 < m'_1 + m'_2 < 4$ et $0 < n'_1 + n'_2 < 4$ pour donner le sel de sodium selon la revendication 1.

**9.** Procédé de stockage d'un sel de sodium représenté par la formule générale (IV) :

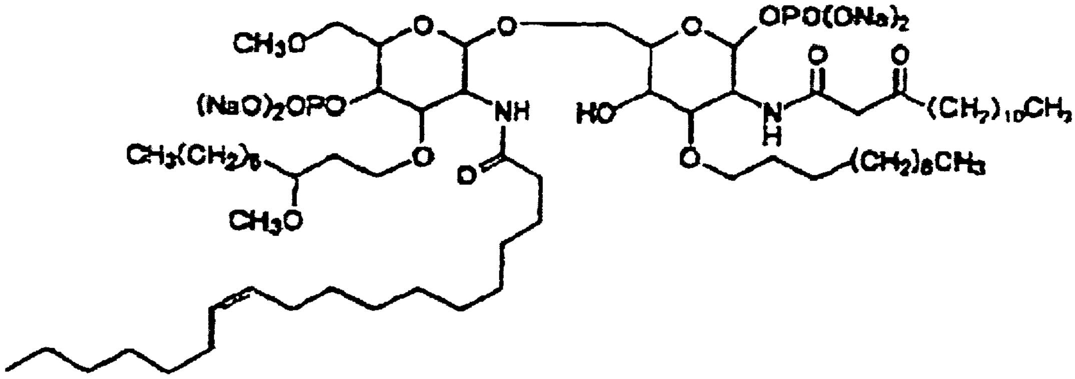

(IV).

**caractérisé en ce qu'**un sel de sodium de formule générale (IV) est fait pour coexister avec un sel de sodium représenté par la formule générale (III) :

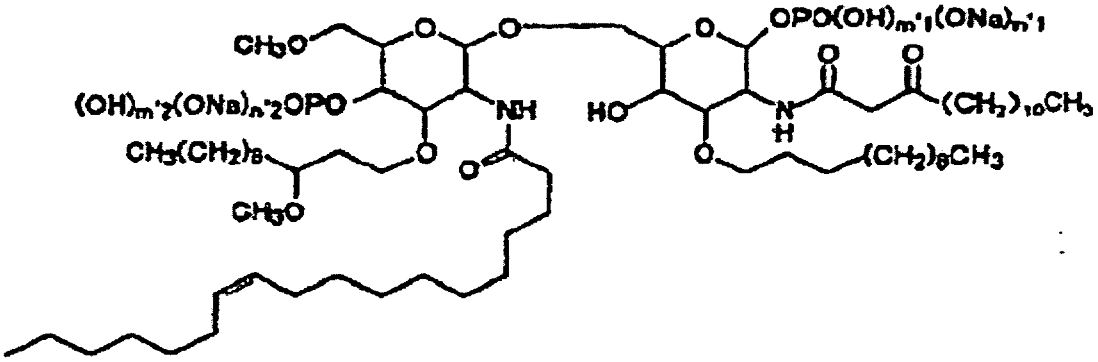

(III)

dans laquelle $m'_1$, $n'_1$, $m'_2$ et $n'_2$ représentent indépendamment 0 ou un entier non supérieur à 2, tout en satisfaisant

à $m'_1 + n'_1 = 2$, $m'_2 + n'_2 = 2$, $0 < m'_1 + m'_2 < 4$ et $0 < n'_1 + n'_2 < 4$ pour donner le sel de sodium selon la revendication 1.

**10.** Formulation selon la revendication 6, dans laquelle la formulation affiche une décomposition moindre par rapport à une formulation comprenant le composé représenté par la formule générale (IV) en l'absence du composé représenté par la formule générale (III).

[FIG. 1]

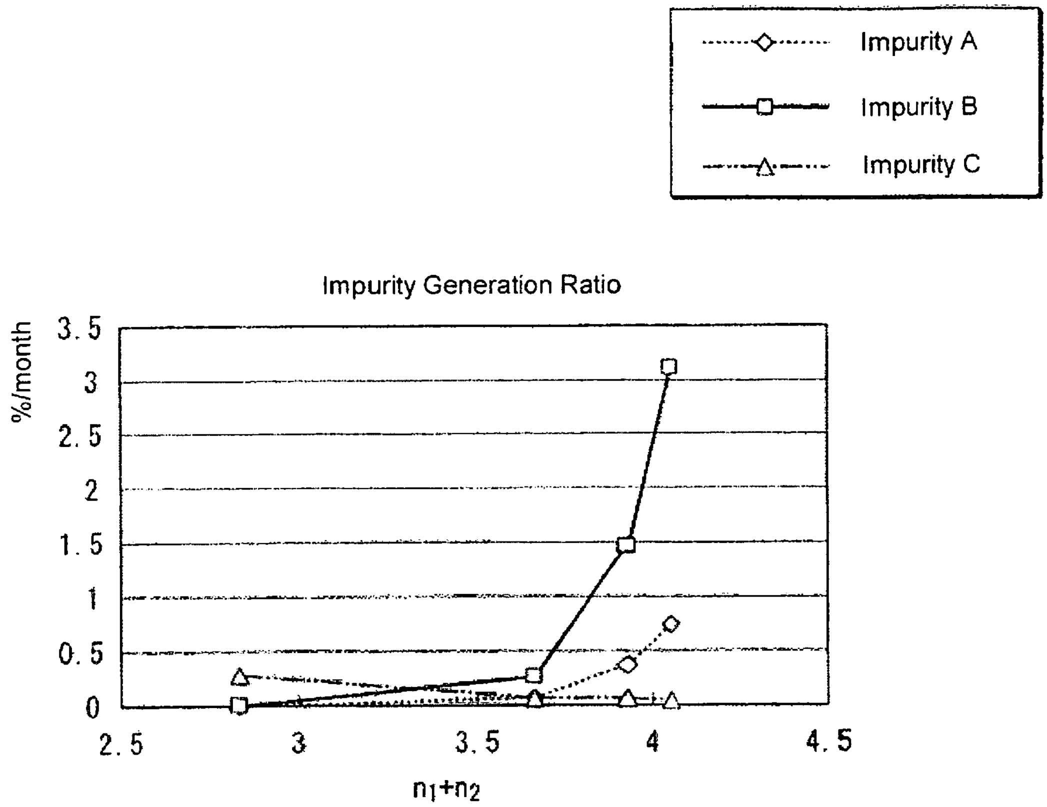
Impurity A
Impurity B
Impurity C
Impurity Generation Ratio
%/month
3.5
3
2.5
2
1.5
1
0.5
0
2.5
3
3.5
4
4.5
$n_1+n_2$

[FIG. 2]

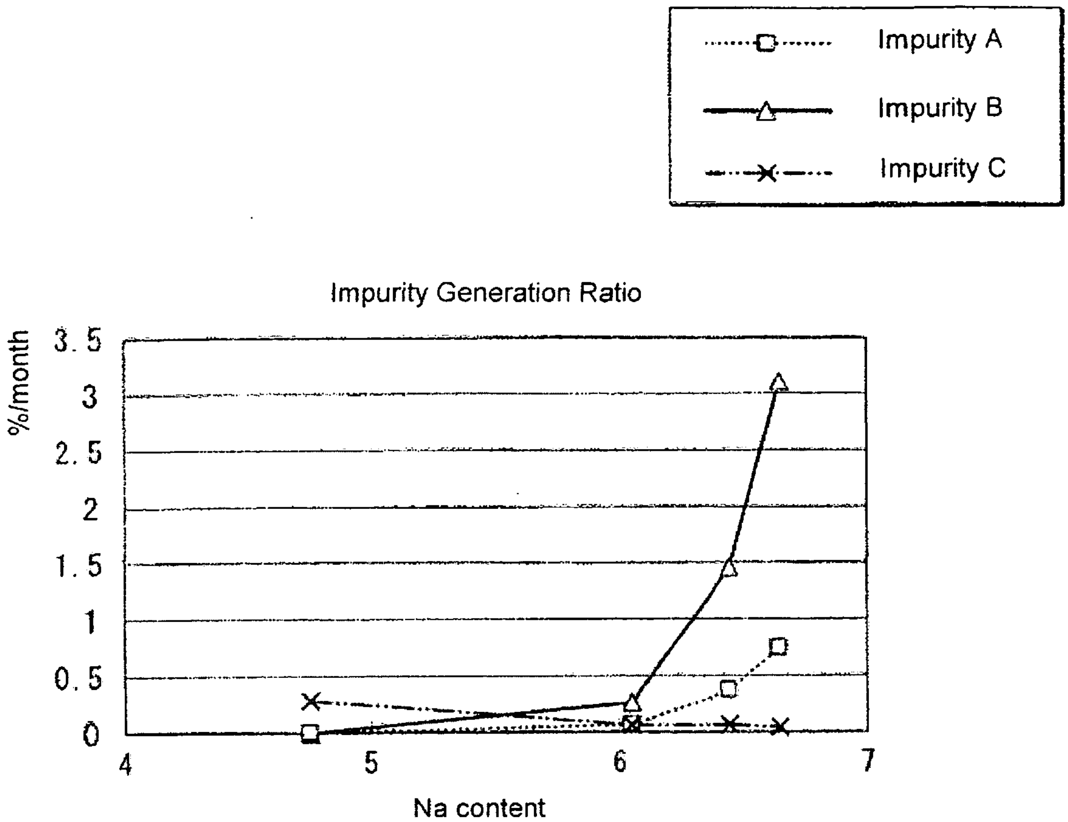
Impurity A
Impurity B
Impurity C
Impurity Generation Ratio
%/month
3.5
3
2.5
2
1.5
1
0.5
0
4
5
6
7
Na content

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006315020 A
- WO 2004071465 A
- WO 9639411 A
- WO 2004074303 A


### Non-patent literature cited in the description


- **LYNN et al.** *J. Pharmacol. Exp. Ther.,* 2004, vol. 308 (1), 175-181